(19) Europäisches Patentamt
European Patent Office
Office européen des brevets

(11) EP 1 310 559 A1

# (12) EUROPEAN PATENT APPLICATION

(43) Date of publication:
**14.05.2003 Bulletin 2003/20**

(51) Int Cl.[7]: **C12N 15/21**, C07K 14/56, C12P 21/02, A61K 38/21

(21) Application number: **02024919.9**

(22) Date of filing: **06.11.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **08.11.2001 IT MI20012345**

(71) Applicant: **International Centre for Genetic Engineering and Biotechnology**
**34012 Trieste (IT)**

(72) Inventors:
- **Tisminetzky, Sergio Gabriel, Int. Centre for Gen. 34012 Trieste (IT)**
- **Baralle, Francisco Ernesto, Int. Centre for Gen. 34012 Trieste (IT)**

(74) Representative: **Albrecht, Thomas, Dr. et al**
**Kraus & Weisert,**
**Thomas-Wimmer-Ring 15**
**80539 München (DE)**

Remarks:
- The sequence listing, which is published as annex to the application documents, was filed after the date of filing. The applicant has declared that it does not include matter which goes beyond the content of the application as filed.
- A request for correction of a part of the description has been filed pursuant to Rule 88 EPC. A decision on the request will be taken during the proceedings before the Examining Division (Guidelines for Examination in the EPO, A-V, 3.).

## (54) Process for the production of alpha interferon of therapeutical degree

(57) The present invention refers to a process for the production of native alpha interferon of therapeutical degree, comprising a step of oxidizing renaturation and only one chromatographic step on cationic exchanger. Further object of the invention is native alpha interferon of therapeutical degree obtained by means of the process of the invention and corresponding to the mature form of the human alpha interferon.

EP 1 310 559 A1

SEQUENCE LISTING

<110> Int. Centre for Genetic Engineering and Biotechn.

<120> PROCESS FOR THE PRODUCTION OF ALPHA INTERFERON OF THERAPEUTICAL DEGREE

<130> 12802EP

<140> 02 024 919.9
<141> 2002-11-06

<150> MI2001A002345
<151> 2001-11-08

<160> 5

<170> PatentIn Ver. 2.1

<210> 1
<211> 499
<212> DNA
<213> Artificial Sequence

<220>
<223> Description of Artificial Sequence:Mature alpha 2b IFN sequence optimized according to the preferential use of the E. coli codons

<400> 1
```
tgtgatctgc cgcaaactca cagcctgggt agccgtcgta ccttgatgct cctggcacag 60
atgaggagaa tctctctttt ctcctgcttg aaggacagac atgactttgg atttccccag 120
gaggagtttg gcaaccagtt ccaaaaggct gaaaccatcc ctgtcctcca tgagatgatc 180
cagcagatct tcaatctctt cagcacaaag gactcatctg ctgcttggga tgagaccctc 240
ctagacaaat tctacactga actctaccag cagctgaatg acctggaagc ctgtgtgata 300
caggggtgg gggtgacaga gactcccctg atgaaggaag gactccattc tggctgtgag 360
gaaatacttc caaagaatca ctctctatct gaaagagaag aaatacagcc cttgtgcctg 420
ggaggttgtc agagcagaaa tcatgagatc tttttctttg tcaacaaact tgcaagaaag 480
tttaagaagt aaggaatga                                              499
```

<210> 2
<211> 49
<212> DNA
<213> Artificial Sequence

<220>
<223> Description of Artificial Sequence:Oligonucleotide

used for the amplification of the 5' of the alpha 2 IFN modified according to the preferential use of the E. coli codons

<400> 2
agctcatatg tgtgatctgc cgcaaaccca cagcctgggt agccgtcgt 49

<210> 3
<211> 28
<212> DNA
<213> Artificial Sequence

<220>
<223> Description of Artificial Sequence:Oligonucleotide used for the amplification of the 3' of the alpha 2 IFN

<400> 3
acgtggatcc tcattcctta cttcttaa 28

<210> 4
<211> 17
<212> DNA
<213> Artificial Sequence

<220>
<223> Description of Artificial Sequence:T7 promoter

<400> 4
taatacgact cactata 17

<210> 5
<211> 19
<212> DNA
<213> Artificial Sequence

<220>
<223> Description of Artificial Sequence:T7 terminator

<400> 5
gctagttatt gctcagcgg 19

## Description

## BACKGROUND OF THE INVENTION

The field of the invention is that of the renaturation and purification of cytokines produced through synthetic or recombinant way for the manufacture of molecules having therapeutical degree.

The interferons are pleiotropic cytokines produced and secreted from different kinds of cells (epithelial cells, fibroblasts, lymphocytes, macrophages) by induction from a series of stimulations (virus, bacteria, cells, tumours and macromolecules) with a series of antiviral, antiproliferative and immune-stimulating functions. Following its endogenous production or its administration, the interferon interacts with the specific receptors on the cells surface and starts the transduction of the signal through the cytoplasm until the nucleus, inducing the expression of genes which codify for specific proteins having antiviral and immuno-stimulating activity.

Five main classes of interferons are described: interferon $\alpha$, $\beta$, $\tau$, $\gamma$ and $\bar{\omega}$. $\alpha$ and $\beta$ interferons, known as type 1 interferons, are structurally correlated, they are stable at acid pH and compete for the same cell-receptor. The proteins that constitute the alpha interferon's family are codified from 14 genes, all located on the chromosome 9.

At present, alpha, beta and gamma interferons can be manufactured under recombinant form with the double advantage of getting much higher amounts of product compared to those obtained through the isolation from natural sources (leukocytes, fibroblasts, lymphocytes) and of reducing the complexity of the processes of purification and check of the safety of the product.

The alpha interferon has been one of the first proteins to be produced by means of *Escherichia coli* with the DNA recombinant technology (Derynck et al., Nature, 287, 193-197, 1980; Nagata et al., Nature, 284, 316-320, 1980). The expression of recombinant proteins in bacteria is preferable because, unlike that in eucaryotic cells or that of the natural products expressed in culture in mammalian cells, like the interferon described by Yonehara S. et al., J. Biol. Chem., 256, 3770-3775, 1981, gives a molecule without contaminants, i.e. retrovirus or serum contaminants of animal origin, not well known or characterized, like for instance the agent of the BSE, potentially present into the serum of bovine origin used for the cellular cultures. The same contamination risks are clearly present in the case of purification by means of immuno-affinity with monoclonal or polyclonal antibodies.

The expression in E. coli anyway shows other problems: it has in fact been seen that the efficient expression of recombinant proteins in E. coli brings to the formation of insoluble aggregates known as inclusion bodies (Taylor G. et al., Bio/Technol., 4, 553-557, 1986; Schein C. Bio/Technol. 8, 308-317, 1990).

The proteins obtained as inclusion bodies are not correctly shaped as to the tridimensionality and therefore they have not an optimum biological activity. To get such proteins under native form it is first necessary a denaturation phase and then a renaturation phase, oxidizing, in case also the disulfide bridges have to be formed like in the natural protein.

Many renaturation methods of recombinant proteins are known in the art: each renaturation protocol is anyway specific for the considered protein and its efficiency depends on the complexity of the protein and on the presence in it of cysteins and disulfide bridges (Rudolph R. & Lilie H., FASEB J., 10, 49-56, 1996; Lilie H. et al., Curr. Opin. Biotechnol., 9, 497-501, 1998; Misawa S. and Kumagai I., Biopolymers, 51, 297-307, 1999).

The processes of oxidation and renaturation are not much efficient: there are remarkable losses of material and some by-products are produced (partially reduced forms, molecules with unnatural intramolecular bridges and oligomers due to the formation of inter-molecular disulfide bridges) which have to be eliminated during the subsequent phases of purification.

In particular, for the products of therapeutic use the purification has to provide for the removing not only of the endotoxins of bacterial origin or of other contaminants stemming from the system of expression, but also of the molecules having wrong three-dimensional structure and therefore totally or partially inactive.

Different and complex methods of production and purification have been developed for the interferons, which however represent a family little homogeneous from the structural point of view, some of which are listed afterwards. The interferon produced in bacteria as inclusion bodies is dissolved by using a 8M solution of guanidine - HCl and then dialyzed with 20 volumes of water at 4°C in: "Purification of Recombinant Human IFN-$\alpha$2", Thatcher D. and Panayotatos N., Methods Enzymol. 119, 166-177, 1986. The resulting solution is charged on a SP-Sepharose A-50 column and the fractions with the interferon are passed through a glass funnel containing DEAE-Sephadex A20. The "flow-through" of this column is submitted to a further step of purification through a Sepharose 6B-Cu column. The fractions containing the interferon are precipitated with $(NH_4)_2SO_4$ and are charged on a Sephacryl S-200 column. The fractions corresponding to proteins having molecular weight between 40 and 60 kDa are precipitated with $(NH_4)_2SO_4$ and further dialyzed.

A process is described in EP 0110302 and US 4432895 wherein the pellet of freeze-dried E. coli is suspended again in water and alkalinized to help the flocculation of the cellular residues. A treatment with Guanidine-HCl follows. The cellular lysate after filtration is charged on an immuno-affinity column obtained with a specific monoclonal antibody covalently bound to the Sepharose 4B matrix. The interferon eluted from the affinity column is submitted to two further steps of purification through a CM-52 column and a Sephadex G-50 column.

A process that uses three sequential chromatographic phases is described in EP 094672. First the interferon-β present in the raw cellular lysate is absorbed on a glass column from which it is eluted by means of a solution containing tetramethylammonium chloride, sodium chloride and propyleneglycol. The eluate from the first column is charged in the subsequent phase on a Sephacryl S-200 column. The fractions corresponding to proteins having molecular weight between 40 and 60 kDa are subsequently charged on a chromatographic column containing a resin with $Zn^{++}$ chelating, to which the interferon does not bind so being purified from the other proteins.

The alpha interferon is produced as fusion protein by using an expression vector of the pGEX (Amersham-Pharmacia) series in Boyer S.J. et al. (J. Biol. Regul. Homeost. Agents, 6, 99-102, 1992). The bacterial product is purified through an affinity column containing Glutathione-Sepharose 4B. The fusion protein is eluted from the column and the interferon is released by means of enzymatic cut with the Factor X.

A precipitation with protamine sulphate, an anionic exchange chromatography and an immuno-affinity column with a specific monoclonal is used in Waine G.J. et al., "Improved production and purification of interferon alpha 4a using a T7 RNA polymerase expression system", Biochem. Int., 28, 255-263, 1992.

A progressive series of chromatographies is used for the purification in: "Large-scale production of recombinant proteins: human leukocyte interferon" of Khan F.R. and Rai V.R., Bioprocess Technol., 7, 161-169, 1990: a carboxymethylcellulose column, a Cu-chelating affinity column, an ionic exchange column and lastly a gel filtration chromatography.

The production of alpha IFN in yeast is described in EP 553494. The dissolved material is purified through a progressive series of chromatographies: an anionic exchange chromatography followed by a cationic exchange chromatography and lastly a gel filtration chromatography.

In US 4485038, the alpha IFN is expressed in bacteria and purified through a first crossing of the raw bacterial lysate through a chromatographic column containing glass (Controlled Pore Glass) or silica acids balanced in phosphate buffer at pH between 6 and 8. The elution is carried out with sodium or ammonium chloride mixed with ethylene glycol and propylene glycol. The eluate, whose pH is balanced at pH 4.5 - 6.0, is first passed through a carboxymethylderivative column and then through a cationic exchange column. The eluate of the last column is charged on a Phenyl-Agarose column and the fractions containing the interferon are eluted with a 50-70% solution of ethylene glycol.

The process described in US 5196323 provides for a progressive series of chromatographies: they start with a column containing cellulose DE-52, then they go on with an immuno-affinity column with an anti-interferon IgG monoclonal antibody and they end with a cationic exchange column.

A process is described in US 4511502 wherein the proteins produced as inclusion bodies are dissolved by means of a solution containing Guanidine 4-9M or Thiocyanate and 0.01-2% detergent. The bacterial lysate is absorbed on a DEAE cellulose column and the interferon is purified by means of a gel filtration chromatography with the Sephacryl S-300 matrix.

A process for the isolation of interferon from inclusion bodies through solubilization with guanidine and subsequent loading of this solution on a column of hydrophobic interaction containing N-butyl-TSK is described in US 4845032. The fractions containing the interferon are eluted with saline solutions having less than 0.1M concentrations. The purification of alpha interferon by using a C-4 reverse phase column, wherein the fractions containing the interferon are recovered by means of a solution made of 0.1% trifluoroacetic acid and 46% $CH_3CN$ is described in US 4765903 (Purification of monomeric interferon. D'Andrea M.J., Tarnowski S.J. and Clark A.D.). The material comes from a previous purification step with an immuno-affinity chromatography. The fractions eluted from the C-4 column are further purified through a cationic exchange chromatography on carboxymethylcellulose.

A process providing for the extraction of the alpha interferon from bacteria by means of a chaotropic agent is disclosed in EP 679718. The subsequent renaturation is carried out by using water or a buffer solution. The alpha interferon is purified through different subsequent chromatographies: metal-chelating chromatography, cationic exchange chromatography, anionic exchange chromatography, gel filtration chromatography.

Also the expression in an eucaryotic system, like the yeast, does not warrant the right three-dimensional structure of the recombinant product.

The IFN-alpha 8 produced in S. cerevisiae has to be denatured and renatured and purified through a Cu-chelating affinity chromatography and an anion exchange chromatography as reported for instance in Di Marco S. et al, J. Biotechnol., 50, 63-73, 1996.

Summing up, the methods for the production/preparation of alpha IFN expect or purifications with almost one immuno-affinity step which presents potential problems of contamination, or, alternately, length processes comprising many chromatographic steps and therefore quite laborious.

Therefore the need for a method of purification of alpha IFN of therapeutical degree which is simple, reproducible and which warrants advantageous yields is highly requested in the sector.

## SUMMARY OF THE INVENTION

A process for the purification of alpha interferon under native conformation and of therapeutical degree from a denaturation mixture which comprises one step of oxidizing renaturation and a sole chromatographic step on a resin with a strong cationic exchanger, preferably selected between sulfopropyl and methylsulfonate is the main object of the present invention.

The chromatographic step takes place at a pH between 5.3 - 5.7, preferably at pH 5.5 ± 0.1.

According to a particularly preferred aspect, the interferon is the human alpha 2 IFN produced by recombinant way. The oxidizing renaturation takes place in a buffer system at pH between 7.5 and 8.5, preferably pH 8.0 ± 0.1 comprising a labilizing agent, preferably L-arginine or salts and derivatives thereof, at a concentration between 0.3 and 0.6 M, a redox pair, preferably made of L-cysteine at a concentration between 0.1 - 10 mM and salts or derivatives thereof and L-cystine and salts or derivatives thereof at a concentration between 0.005 - 0.5 mM, and optionally a chelating compound. According to a preferred fulfilment, the process comprises a step of de-salting carried out by means of one of the following methods: dialysis, ultrafiltration, gel filtration or dia-filtration, carried out before the chromatographic step.

According to a further aspect, the invention refers to any method of production of alpha interferon of therapeutic degree that comprises the step of oxidizing renaturation and of chromatography on strong cationic exchanger, according to the main object of the invention.

According to a particularly preferred fulfilment, the production of alpha IFN takes place by recombinant way, by means of: 1) intracellular expression of a sequence of DNA codifying for the alpha interferon in an organism preferably procaryote genetically modified, still more preferably E. coli; 2) recovery of the alpha interferon under non-native form, preferably from the inclusion bodies; 3) denaturation of the alpha interferon by means of chaotropic agents, to be subsequently purified by means of the steps a) and b) of the process according to the invention.

According to a further aspect, therefore, the invention refers to human alpha 2 interferon under native conformation and of therapeutical degree, preferably recombinant, obtainable according to the process of the invention, and having a purity degree higher than 99% as measured by means of denaturation gel electrophoresis, carried out according to the main lines of the European Pharmacopoeia. A further aspect of the invention therefore refers to the therapeutical use of the interferon according to the invention.

## DESCRIPTION OF THE FIGURES

**Figure 1. Electrophoresis on reducing SDS-PAGE of the process of production of alpha interferon according to the invention.**

The electrophoresis has been carried out on polyacrylamide gel.

Lane 1: raw bacterial extracts at the end of the fermentation process: the stripe of the mainly expressed protein is alpha interferon, which runs together with the standard preparation of IFN-$\alpha$2 present in lanes 7 and 8, respectively charged with 5 and 25 μg of the lot 2 CRS Cat. No. I0320301 EDQM - European Pharmacopoeia.
Lane 2: supernatant after centrifuging of the inclusion bodies.
Lane 3: washings of the inclusion bodies.
Lane 4: denaturation mixture after re-suspension of the inclusion bodies in GuHCl.
Lane 5: renaturation mixture.
Lane 6: IFN$\alpha$ purified by means of chromatographic separation on cationic exchange.
Lanes 7 and 8: 5 and 25 μg respectively of the lot 2 CRS Cat. No. 10320301 EDQM - European Pharmacopoeia.

**Figure 2. Chromatographic profile of the renaturation mixture after diafiltration on IEC SP 2825 column (Shodex Waters).**

Panel a): abscissas: retention time,
ordinates: conductivity in mSiemens/cm;
Panel b): abscissas: retention time,
ordinates: percent volume of the solution B (0.5 M NaCl).

The renaturation mixture diafiltrated and concentrated and containing the renatured IFN was charged on IEC SP2825 column, balanced at pH 5.5 with sodium acetate 50 mM at a 8.0 ml/min flow. The column was washed with 3 column volumes (CV = 154 ml) using the same balancing buffer. The elution of the native alpha IFN was carried out by means of a linear gradient 10% - 40% of 0.5 M NaCl (solution B) in pH 5.5. buffer of sodium acetate 50 mM in 5 CV. The chromatographic profile shows a main peak (C) corresponding to the IFN-α2 in native conformation and two lower peaks (A) and (B), corresponding to monomeric non-native forms.

**Figure 3. SDS-PAGE under non-reducing (panel a) and reducing (panel b) conditions of the preparation of alpha interferon purified according to the invention, in agreement with the European Pharmacopoeia, Monograph 1997:1100, pages 1031-1035, 1997.**

The proteins were coloured with silver coloration. The values of the molecular weights of the marker are shown beside the gel (Molecular Weight Markers: Cat. No. 7708S, N.E. Biolabs).

Lane 1: markers of molecular weight;
Lanes 2 and 3: preparation of IFN-α2 purified according to the invention, charged respectively in amounts equal to 5 and 25 μg;
Lanes 4-8: alpha interferon standard (lot 2 CRS Cat. No. 10320301, EDQM-European Pharmacopoeia), respectively charged in amounts corresponding to 31.25, 6.25, 1.25, 0.25, 0.05 μg for each lane.
Lane 9 and 10: cellular extracts of the strain of E. coli used for the expression of the recombinant protein transformed with the sole plasmid were charged.

The presence and the percent of the contaminants in the preparation of IFN-α2 purified according to the invention, in particular of stripes having molecular weight lower or higher than that of the IFN-α2 (corresponding to about 20kDa), was evaluated by comparing the intensity of said stripes with reference to the dilutions of the standard preparation of IFN-α2.
Stripes of molecular weight lower than that of IFN-α2 (presumably corresponding to degradation products) were evaluated in the electrophoresis under reducing conditions (panel b), while the presence and intensity of stripes of molecular weight higher than that of IFN-α2 (presumably corresponding to dimers) were evaluated in the electrophoresis under non-reducing conditions (panel a), according to what disclosed in example 4 and in agreement with the European Pharmacopoeia.

**Figure 4. Immunoblotting of the preparation of purified alpha 2 interferon.**

Panel a) developed with antibodies anti-E. coli for the detection of possible bacterial contaminants.
Panel b): Immunoblotting developed with anti-interferon antibody for the immunological identification of the purified product.

**Panel a):**

Lane 1: markers of molecular weight;
Lanes 2 and 3: preparation of IFN-α2 according to the invention, charged in amounts corresponding to 5 and 25 μg;
Lanes 4-8: preparation of IFN-α2 standard (European Pharmacopoeia) respectively charged with 31.25, 6.25, 1.25, 0.25 and 0.05 μg;
Lanes 9 and 10: total proteins of E. coli respectively charged in amounts corresponding to 10 and 1 μg. The proteic concentration on the extracts was evaluated by means of a commercial kit (Sigma Cat. No. 690-A). The total proteins of E. coli were obtained from a standard fermentation of the BL21 strain transformed with the sole vector (not expressing the IFN), charged as positive control of the antibody reaction. Positive stripes for the anti-E. coli antibody are not noticeable both in the product purified according to the invention and in the standard of the European Pharmacopoeia. Antibody anti-E. coli (Cat. No. RV 1001, Virostat).

**Panel b):**

Immunoblotting developed with anti-interferon antibody. Lanes as in panel a). Stripes corresponding to proteins immunologically similar to IFN-α2 are not noticeable, as expected, in the extracts from the unchanged BL21 strain of E. coli, while a stripe of the molecular weight expected for the alpha interferon (19.3 kDa) is present both in the preparation of IFN-α2 according to the invention and in the standard of the European Pharmacopoeia.
Anti-interferon antibody (Cat. No. AB1434, Chemicon Int. Inc.). Anti-rabbit conjugate: Alkaline Fosfatase conjugate Cat. No. 5373B, Promega. Development system: NBT-BCIP Cat. No. 169-747-1, Boheringer Mannheim. Support: membrane of nitrocellulose Cat. No. 12807-41BL, Sartorius A.G..

**Figure 5. Isoelectrofocusing carried out according to the European Pharmacopoeia, 1997, of the alpha 2 interferon prepared according to the invention, and of the reference standard.**

Panel a): gel coloured with Acid Blue 83R: Coomassie Brillant Blue R-250, Cat. No. 27816, Fluka.

Lane 1: markers of isoelectric point (Broad pl-kit, pH 3.5-9.3, cat. N° 17-0471-00, Amersham Pharmacia Biotech);
Lane 2: reference standard: IFN-$\alpha$2b, lot 2 CRS Cat. No. 10320301, EDQM - European Pharmacopoeia (EP);
Lane 3: IFN-$\alpha$2 according to the invention, renatured in RB1 buffer (see example 3);
Lane 4: IFN-$\alpha$2 renatured in RB2 buffer (see example 3);
Lane 5: IFN-$\alpha$2 renatured in RB3 buffer (see example 3).

It is possible to see how the main stripe of the three different preparations of IFN-$\alpha$2 (lanes 3, 4, 5) runs in position corresponding to the main stripe of the reference standard (lane 2).
Panel b): Graph for the interpolation of the isoelectric point of the preparation of IFN-$\alpha$2 according to the invention. The distances of migration of the markers of reference isoelectric point are reported on the abscissas of the graph and their isoelectric point on the ordinates. The values of the isoelectric point of the samples and of the reference standard (EP) have been interpolated on the line made with such points. The pl values obtained and their compliance to what requested from the European Pharmacopoeia are described in Example 4.

**Figure 6. Chromatographic profile of the interferon according to the invention and of the standard IFN on liquid chromatography in inverted phase.**

Panel a): Alpha 2 interferon according to the invention and panel b) standard alpha 2 IFN of the European Pharmacopoeia. The analysis has been carried out according to the guidelines of the European Pharmacopoeia in order to evaluate the purity of the preparation of the IFN. The chromatogram shown in the panel a) reveals the presence of a main peak having an area corresponding to 98.81% of the total area of the chromatogram and having a retention time on the column equal to 54.56 minutes. The area of the peak corresponding to the standard preparation of IFN-$\alpha$2 (see panel b) results in its turn to be equal to 97.45% of the total area and has a retention time very similar (54.66 minutes).
Other parameters measured in the chromatogram a) for the main peak (IFN according to the invention) are: area mAU*min = 12.5709 and peak height = 25.969 mAU. The corresponding values in the panel b) concerning the preparation of standard IFN are: area mAU*min = 6.4315 and peak height = 14.219 mAU.
Materials and reagents: HPLC: AKTA purifier 10, Cat. No. 18-1400-00, Pharmacia. Column: Lichrospher WP300 RP-18, 4.6x250 C18, 5um, Cat. No. 50137, Merck. Acetonitrile: Hypersolve, Cat. No. 152516Q, BDH. Trifluoroacetic acid: Cat. No. 108262, Merck. Standard IFN: alpha 2b interferon CRS Cat. No. I0330301. European Pharmacopoeia.

**Figure 7. Tryptic map of the alpha 2 interferon according to the invention and of the standard IFN, in accordance with the European Pharmacopoeia.**

The chromatogram obtained by means of liquid chromatography after digestion with trypsin of the preparation of purified interferon according to the invention is shown in the upper part of the figure; that of the EP standard is shown in the lower part.
The two chromatograms, easily comparable visually by specularity, result to be qualitatively similar, as requested from the European Pharmacopoeia.

**Figure 8. Mass spectroscopy of the alpha 2b interferon according to the invention and of the standard alpha 2b interferon carried out by means of IonSpray (PE SCIEX, API 150EX, Perkin Elmer).**

The mass values obtained with the alpha IFN purified according to the invention are shown in the panel a), while those corresponding to the standard preparation are shown in the panel b).
The samples were introduced by means of direct infusion with "positive mode", on the m/z 400-3000 interval. The spectrum deconvolution has been carried out by means of the Biotoolbox software (Applied Biosystem).

## DETAILED DESCRIPTION OF THE INVENTION

The efficient expression of recombinant IFN, like that obtained in prokaryotic organisms, often is obtained to the detriment of a right folding of the protein. Insoluble aggregates, known as inclusion bodies, grow up at intracellular level in E. coli because of the precipitation of denatured forms of the heterologous protein, which in such a way succeeds in constituting also the 30% of the total proteins of the bacterium. The protein also advantageously being under half-pure form has not a native conformation in the inclusion bodies. The inclusion bodies have to be solubilized by denaturation with a chaotropic agent and then submitted to a renaturation step in vitro, that allows the right folding of the protein and the formation of right disulfide bridges, in case they are present, in order to get the protein under native form.

A protein having not only the aminoacid sequence but also three-dimensional conformation equal to that of the natural protein is intended as native form of a recombinant protein.

Many in vitro renaturation protocols exist starting from mixtures wherein the protein is present under partially or totally denatured form. The renaturation protocols and the related yields are very variable and depend on the characteristics of the particular interesting protein, on the number of disulfide bridges present in the natural protein, on its natural aptitude to aggregate, etc..

A process for the purification of alpha IFN under native conformation and of therapeutical degree, from a denaturation mixture essentially comprising a step a) of oxidizing renaturation and a sole step, step b, of chromatography on cationic exchanger, and the relating elution, constitutes the object of the present invention. The mature alpha IFN protein (like that identified under SwissProt with numbers P01563, P01562 and P32881 and under GenBank with the number NM-000605) under monomeric form, not glycosylated, and having two disulfide bridges is intended for alpha interferon under native form.

According to a preferred fulfilment the alpha IFN is of recombinant origin, but it may be produced also by chemical synthesis and with the hydrosulphide groups under reduced form.

In the present invention every aqueous solution containing alpha interferon at a proteic concentration lower than 15 mg/ml, better if comprised between 6-14 mg/ml, still more preferably comprised between 8-12 mg/ml and wherein the interferon is under denatured form because of the presence of a chaotropic agent, like urea or guanidine at a concentration between 4 and 8 M in a buffer system at pH between 7 and 9, preferably pH 8.0±0.2, still more preferably pH 8.0±0.1 and wherein the protein has the hydrosulphide groups under reduced form, is intended as denaturation mixture.

The denaturation mixture may in case comprise also a reducing agent, like for instance DTT (dithiothreitol) or β-mercaptoethanol at a concentration lower than 10 mM.

The chaotropic agent preferably is GuHCl at a concentration between 5 and 7M, preferably 6M, in a buffer system like Tris at 50-150 mM concentration and at pH between 7.5 and 8.5, preferably pH 8.0±0.2, still more preferably pH 8.0±0.1, and moreover it may comprise a chelating agent, like EDTA, preferably at a concentration between 0.1 and 5 mM, still more preferably between 1 and 3, or preferably 2 mM.

Under these conditions the proteic aggregates submitted to mechanic stirring, for instance with a magnetic stirrer at 200 rpm, are completely dissolved. The possible presence of particulate, due to a not complete solubilization of the proteic aggregates may be eliminated before the renaturation phase, for instance by means of centrifugation.

The oxidizing renaturation, corresponding to the phase a) of the process, takes place by slow addition of the denaturation mixture, for instance by dripping, to a renaturation solution, until to reach an end concentration of total proteins (mainly or almost totally made of alpha IFN) comprised between 50 and 300 μg/ml, still more preferably 100-250 μg/ml.

The renaturation solution comprises a labilizing agent and a redox pair and it is buffered at a pH between 7.5 and 8.5, preferably pH 8.0±0.2, still more preferably pH 8.0±0.1, for instance with Tris. Also a chelating agent, like EDTA, can optionally be present preferably at a concentration between 0.1 and 5 mM, still more preferably between 1 and 3, or preferably 2 mM.

A molecule or a compound able to be against the development of macromolecular aggregates of proteic nature in aqueous solution, keeping the proteins under monomeric form and helping the formation of a native conformation, also through a stabilizing/solubilizing action of the renaturation intermediates is intended as labilizing agent, action hypothesized for the arginine (Lilie H. et al., Curr. Opin. Biotechnol., 9, 497-501, 1998). Under such sense also slightly denaturing solutions, like for instance guanidine at a concentration between 0.3-1 M, are labilizing; whereas a system having functional groups able to be reversibly reduced and oxidized within a biological system is intended as redox pair.

L-arginine or salts or derivatives thereof, at a concentration between 0.3 and 0.6 M, preferably between 0.4 and 0.55 M, also more preferably 0.5 M, is preferred among the labilizing agents.

Moreover a redox pair is present within the renaturation solution, preferably made of thiolic reagents having low molecular weight under oxidized and reduced form, like: glutathione under reduced and oxidized form (GSS/GSSH), the cystine/cysteine pair, the cystamine/cysteamine pair, the hydroxyethyl-sulphoxide/2-mercaptoethanol pair. The redox pair cystine/cysteine or salts and derivatives thereof is particularly preferred, respectively at a concentration between 0.1-10 mM for L-cysteine and between 0.005-0.5 mM for L-cystine and/or salts or derivatives thereof. Still more preferably such concentrations are between 0.5 - 2 mM for L-cysteine or salts and derivatives thereof and between 0.03 - 0.07 mM for L-cystine.

The pH of the renaturation mixture is kept at a value between 7.5 and 8.5, preferably 8.0 ± 0.2, still more preferably 8.0 ± 0.1, after the addition of the denaturation mixture to the renaturing solution. The oxidizing renaturation of the IFN-α in the renaturation mixture takes place at a temperature between 5°C and 25°C preferably between 16°C and 24°C, still more preferably 22°C, preferably for a time between 18 and 50 hours, preferably 40 hours.

The amount of product renatured in native conformation can be temporally checked during the renaturation phase, for instance through HPLC in reverse phase or through other methods known to the skilled man.

According to a particularly preferred fulfilment, the phase of oxidizing renaturation, corresponding to the phase a) of the process, is followed by a phase of de-salting that can be carried out for instance by means of dialysis, according to methods known to the skilled man, or by means of ultra-filtration, gel filtration, dia-filtration by means of ultra-filtration, with the aim to remove the labilizing/denaturing agent, and to lower the ionic concentration of the renaturation mixture. Optionally, and in addition to such treatment, a gel filtration is executed and the proteic material can be concentrated before the chromatographic step b).

The renaturation mixture, de-salted, containing both the renatured alpha IFN, (i.e. under native conformation), and that not renatured (under non-native conformation, like for instance that under dimeric form, or under not completely oxidized form), is then brought at a pH between 5.3 and 5.7, preferably pH 5.5±0.1, by means of a concentrated acid, preferably glacial acetic acid. Optionally the solution can be filtered on 0.45 μm filters, or centrifuged, in order to remove possible precipitates due to the acidification.

Subsequently the de-salted renaturation solution, optionally concentrated, and acidified, is charged on a chromatographic column having a strong cationic exchanger as functional group, according to the phase b) of the process. Commercially available resins are used for the chromatographic column having the following characteristics: the functional group has to be a strong cationic exchanger selected from the sulfopropyl or the methylsulfonate group, preferably on a matrix having the size of the particles lower than 25 μ. The column IEC-SP 2825 (Shodex, Waters), which uses a polyhydroxymethacrylate matrix with sulfopropyl functional group, is particularly preferred. Alternately a Source 15S resin is used, having a polystyrene matrix, cross-linked with divinylbenzene (Pharmacia cat. N. 17-0944-01), having the methylsulfonate as functional group.

The chromatographic separation takes place in buffer solutions having pH between 5.3 and 5.7, preferably pH 5.5±0.1. The buffer solution is preferably acetate buffer, at a concentration between 10 and 80 mM, preferably 50 mM. The chromatographic column is preferably balanced before the charge of the sample with the same buffer of charging having pH between 5.3 and 5.7, still more preferably pH 5.5±0.1. Other buffer solutions can anyway be used, as known in the field, provided that a pH value between 5.3 and 5.7 is kept, preferably pH 5.5±0.1. After charging the sample, the column is treated in order to eliminate aspecific ligands for instance by washing with many volumes of the same charging buffer, as known in the field.

The alpha IFN under native conformation is eluted from the cationic exchange resin by charging an elution buffer containing a linear saline gradient from 0 to 1 M of a monovalent salt, still more preferably NaCl at a concentration between 0 and 500 mM. The elution buffer is preferably acetate buffer with the same characteristics as that used for charging the sample and for balancing the column.

According to this preferred fulfilment, the linear saline gradient is made by mixing an acetate buffer solution, preferably sodium acetate at a concentration between 10 and 80 mM, preferably 50 mM, having pH between 5.3 and 5.7, preferably pH 5.5±0.1, with an identical solution containing in addition the monovalent salt, preferably sodium chloride, at 1M concentration, or more preferably between 0.4 and 0.6 M, still more preferably 0.5 M, in the same acetate buffer.

The elution of the native alpha IFN takes place by charging on the column a 10%-40% linear gradient of a monovalent salt, preferably 0.5 M NaCl, in a pH 5.5±0.1 buffer solution, preferably made of 50 mM sodium acetate in a volume between 4 and 12 column volumes, still more preferably between 5 and 10 CV (CV = column volumes), at an ionic concentration corresponding to a conductivity value between 14 and 16 mSiemens/cm.

The chromatographic profile obtained and shown in figure 2 reveals a main peak (c) corresponding to IFN-α2 under native conformation, and two smaller peaks (a) and (b), corresponding to monomeric non-native forms.

Under these conditions, the main peak of the chromatography, corresponding to monomeric IFN-α under native conformation, can eluate at a concentration between 0.3 and 0.6 mg/ml, and can be directly sterilized by filtration.

Alternately, the elution of the native alpha interferon is isocratically obtained by means of a saline solution having a conductivity between 5 and 7 milliSiemens/cm, preferably 6 milliSiemens/cm, corresponding to the 4% of a 0.5 M NaCl solution in acetate buffer. The main peak, corresponding to the preparation of alpha interferon under native conformation, elutes between 18 and 26 column volumes.

After the steps a) and b) the purification process optionally comprises a step c) of molecular exclusion chromatography or of gel filtration, through which the alpha IFN under native conformation can be concentrated in the buffer more suitable for the subsequent formulations. This step takes place on commercially available chromatographic matrices, like for instance the Sephacril S-75 or Superdex 75 (Pharmacia Biotech) resins, previously balanced with the more suitable buffer. The peak eluted from the gel filtration column can be subsequently treated according to the use, for instance sterilely filtered and concentrated.

The process according to the invention is particularly advantageous because it comprises a sole chromatographic step, corresponding to the point b) of the process, for the purification of the alpha IFN under native conformation and of therapeutical degree.

The complete chromatographic separation of the non-native forms of the alpha interferon, and therefore its uttermost purity, is made possible because of uttermost efficiency of the oxidative renaturation step a) which is, in step a) of the process according to the invention, higher than 50%, still more preferably higher than 55%, in respect of the whole interferon present within the renaturation mixture, and because of the characteristics of the chromatographic column.

According to a further aspect, the invention therefore spreads to the alpha IFN product of therapeutical degree, in agreement with the definition and the requirements of the European Pharmacopoeia, obtained with the process comprising steps a) and b) of the present invention.

Such alpha interferon is preferably recombinant and human, still more preferably of type 2 (a and b) and has a purity degree higher than 98%, still more preferably higher than or equal to 99%, according to the analyses carried out according to the Monography on "Alpha 2 interferon concentrated solution" 1110: 1031-1035, 1997 (to which it is referred for all the measurements carried out according to it and often for the sake of brevity quoted as Monography on alpha interferon) in particular according to the electrophoretic analysis on denaturing polyacrylamide gel (SDS-PAGE) under reducing and non-reducing conditions, coloured with silver coloration.

The analyses of identity, purity and activity carried out according to the guidelines of the "European Pharmacopoeia" show an absolute superimposition of the product obtainable with the process according to the invention with the reference standards: Alpha 2 interferon CRS (IFN-alpha 2a, lot 1, cat. N° 10320300 and IFN-alpha 2b, lot n°2, cat. N° I0320301).

The chemical-physical analyses provided from the European Pharmacopoeia (Monography on "Alpha 2 interferon concentrated solution" 1110: 1031-1035, 1997) comprise: a denaturing electrophoresis under reducing and non-reducing conditions, the determination of the isoelectric point by means of isoelectrofocalization, the peptidic map after tryptic digestion, HPLC under reverse phase, the determination of the biological power and of the endotoxins content, in comparison with one or more reference standards, are described in full in Example 4.

The characteristics that make of therapeutical degree the product according to the invention after an oxidizing renaturation step and a sole chromatographic step, are obtained according to the known prior art only with long and complex processes that comprise many chromatographic steps. The yields of the purification process are increased very often, according to the known prior art, by using immuno-affinity columns that anyway show the drawbacks related to the use of antibodies and to the possibility of contamination of the end product.

The process according to the invention is therefore a remarkable progress in the field, because it joins the simplicity of fulfilment, tied to a sole chromatographic step, to a yield higher than 60 mg of alpha IFN under native conformation and of therapeutical degree for each litre of bacterial culture and to an utmost purity of the end product, higher than 98%, preferably than 99% for the therapeutical use.

The biological activity of the product according to the invention is very high and anyway not lower than 2.5 x $10^8$ IU/mg, still more preferably higher than 3 x $10^8$ IU/mg.

Also the endotoxins content of the alpha IFN eluted from the gel filtration column according to the process of the present invention, corresponds to the values required from the European Pharmacopoeia. The values measured are even lower than 25 IU minimum value within the volume containing one mg of protein, well less than the limit permitted according to the European Pharmacopoeia (Monography on Alpha 2 interferon concentrated solution 1110: 1031-1035, 1997) that has been fixed at 100 IU of endotoxin for the volume containing 1 mg of protein.

The alpha IFN obtained according to the process of the invention is stable for one year, also in absence of particular formulations, without clearly visible alterations of the characteristics of homogeneity and of biological activity as shown by biological and biochemical tests (HPLC and SDS-PAGE).

The human alpha IFN is particularly preferred, still more preferably of type 2 (a and b), comprised its allelic variants or mutations, independently from its manufacturing process, even if it is preferably obtained by recombinant way.

According to a further aspect therefore the invention spreads to whichever manufacturing process of the alpha interferon under native form wherein the alpha interferon, preferably human, is produced by expression as recombinant protein, denatured according to methods known in the art and subsequently renatured according to step a) of the process of the invention and then purified by cationic exchange chromatography, as described for step b) of the process according to the invention.

In the preferred fulfilment, connected with the expression of the protein in recombinant systems, the production of alpha IFN takes place through a process comprising the following steps, carried out before steps a) and b):

1) intracellular expression of a sequence of DNA codifying for the alpha IFN in a prokaryotic organism or in a simplex eukaryote;
2) recovery of the alpha interferon under inactive or partially active form;
3) treatment of the alpha interferon under inactive form with concentrated solutions of chaotropic agents.

At item 1), the sequence of alpha IFN is preferably of type 2.
A mixture of recombinant alpha IFN under denatured or partially denatured form suitable for the oxidizing renaturation according to the item a) of the process according to the invention is obtained through the steps 1-3.
The expression of recombinant alpha interferon within unicellular organisms genetically modified is carried out according to methods known in the art for the production of heterologous proteins. Bacterial microorganisms like for instance E. coli, B. subtilis, or yeasts like S. cerevisiae, P. pastoris, K. Lactis, etc. can be used. The E. coli bacterium transformed by means of an expression vector containing a strong promoter before the sequence codifying for the alpha interferon is particularly preferred. Strong promoters used in the bacteria are known in the art: some examples are the promoter trp, lac, tac, trc, $P_L$ or of the T7 polymerase (see: Molecular Cloning "A Laboratory Manual" $2^{nd}$ Edition, Sambrook J., Fritsch E.F. and Maniatis T., Cold Spring Harbor Laboratory Press, 1989). A constitutive or inducible expression of the interesting protein at levels than can arrive to form the 50% of the total proteins of the bacterium can be obtained through them.
The E. coli strain is preferably protease negative, for instance omp (outer membrane protease) or lon negative, and still more preferably it is the BL21 strain (Grodberg J. and Dunn J.J., J. Bacteriol., 170, 1245-1253, 1988). The expression of the recombinant protein can be inducible or constitutive. The expression vectors that can be used for the intracellular expression in E. coli are known in the field and can be used in the present invention provided that they lead to the expression of the heterologous protein in amount higher than 30% of the total proteins of E. coli and that their presence can be selected.
The codifying sequences of many mammalian species for the alpha IFN are known in the literature and are collected in databases of nucleotide sequences, for instance in GenBank (www.ncbi.nlm.nih.gov/Genbank). They can be optimized according to the preferential use of the codons (codon usage) of the organism in which the expression takes place. The sequence codifying for the human alpha interferon, in particular of type 2, still more preferably IFN alpha 2 (a and b), identified under the number NM_000605 in the database, optimized according to the codon usage of E. coli, i.e. in agreement with the most frequently used codons in this organism, is particularly preferred. This preferred fulfilment is carried out according to the suggestions of J. Michael Cherry and by using the GCG "Codon Frequency" program (ftp://ftp.es.embnet.org/pub/databases/codonusage/eco.cod). The nucleotide sequence codifying for the alpha 2b IFN, corresponding to the mature form of the human protein, optimized according to the codon usage of E. coli, is shown in the enclosure "Sequences list" as seq IDN1, placed at the bottom of the patent application. The sequence codifying for the alpha 2a IFN can be derived and expressed from the identifying number in GenBank and according to the optimization program for E. coli, that shows, with respect to the interferon 2b, one lysine in position 23 instead of one arginine, or for the interferon 2c, which shows one arginine in position 23 as well as in position 34, instead of one histidine present both in alpha 2a and 2b IFN and for all their allelic variations and conservative mutations (aminoacidic numeration referred to the mature product). However all the DNA sequences codifying for the alpha interferon, both of synthetic and natural origin, can be used for the production of recombinant IFN.
According to this preferred fulfilment, the E. coli strain transformed with the vector containing the nucleotide sequence codifying for the mature alpha IFN, is grown in a culture medium, like LB (Luria-Broth) or TB (Terrific-Broth) containing an antibiotic whose resistance is codified from the expression vector, preferably kanamycin, for a period of time between 6-8 and 20 hours, according to methods known to the skilled man (Molecular Cloning "A Laboratory Manual" $2^{nd}$ Edition, Sambrook J., Fritsch E.F. and Maniatis T., Cold Spring Harbor Laboratory Press, 1989). The bacteria are recovered at the end of the fermentation for instance by centrifuging at 2,600 rpm, they are washed for instance with water or with an aqueous solution and resuspended in water, or in an aqueous solution, in ratio of about 10-20, preferably 15 g, of wet weight of bacteria for 100 ml of water, and are lysed or broken for instance by sonication or by treatment under high pressure, or by means of other tools apt to recover the inclusion bodies known to the skilled man. The inclusion bodies are recovered by centrifugation (20,000 - 22,000 rpm) optionally washed with water and resuspended in a some volume of water. A reducing agent, like for instance β-Me or DTT at a concentration lower than 10 mM, can be added to the aqueous solution in some circumstances.
The proteic insoluble fraction is then suspended again at an end proteic concentration between 6 and 14 mg/ml, in a denaturing aqueous solution comprising a chaotropic agent, like for instance urea or guanidine, at high concentration, for instance between 4 and 10 M. The guanidine or salts thereof, at a concentration between 5 and 8 M, still more preferably 6 M, is particularly preferred among the chaotropic agents. The denaturing solution comprises a buffer system, like for instance Tris at a concentration between 10 and 150 mM, preferably 50 mM, and having a pH between 7.0 and 9.0, preferably pH 8.0±0.1. A chelating agent, like EDTA, at a concentration between 0.01 and 5 mM, preferably 2mM, is optionally present.

Usually, an optimum proteic concentration in the denaturation solution, i.e. between 6 and 14 mg/ml, is obtained by adding from 15 to 25 ml of denaturing solution for each wet gram of included bodies or of insoluble fraction. The solution is left under stirring for at least 15', preferably at least 30' or until the insoluble fraction is dissolved, at room temperature.

The incompletely resuspended particulate can be removed at the end of the denaturation treatment for instance by centrifugation, and then the denaturation solution containing the alpha interferon under non-native (denatured) form is submitted to a purification process comprising a renaturation treatment corresponding to the step a) of the process and then to a chromatographic step, corresponding to the step b) of the process according to the invention.

The invention is hereinafter exemplified in the following experimental examples, which anyway have no restrictive scope of the extent of the invention.

## EXPERIMENTAL SECTION

Example 1

Cloning of the gene codifying for the human alpha 2b interferon

The DNA sequence codifying for the human alpha 2b interferon was isolated from human DNA obtained starting from lymphocytes purified from peripheral blood according to the procedure described in Molecular Cloning "A Laboratory Manual" 2nd Edition, Sambrook J., Fritsch E.F. and Maniatis T., Cold Spring Harbor Laboratory Press, 1989, slightly modified. Shortly, the lymphocytes were suspended again in 0.5 ml of a lysis buffer (10 mM Tris - HCl pH8 , 10 mM EDTA, 50 mM NaCl, 2% SDS), to which Proteinase K at the end concentration of 0.3 mg/ml (15 µl of a 10 mg/ml solution) was added. The solution was incubated for one hour at 56°C and then 0.5 ml of a solution of Phenol - Chloroform - Isoamyl Alcohol (25:24:1) saturated with 50 mM Tris-HCl pH 8 were added by stirring for one minute. After 5 minutes of centrifugation at 13,000 rpm the aqueous layer is transferred in a clean tube and the extraction with the solution of Phenol - Chloroform - Isoamyl Alcohol is repeated until the aqueous layer looks clear.

At the end the remaining traces of phenol in the aqueous layer were removed by washing with one volume of n-butanol and still recovering the aqueous layer. The amount of purified human DNA was spectrophotometrically evaluated by using the following conversion value: the absorbance at 260 nm of a DNA solution at a concentration equal to 50 µg/ml is 1 OD).

By using the so purified genomic DNA as matrix, the DNA codifying for the mature 2b interferon was amplified by means of the use of the thermoresistant Taq DNA polymerase and of oligonucleotides specific for the gene of the interferon (PCR) according to the following procedure.

The following reagents were mixed in a 0.5 ml Eppendorf tube:

- 50 µl of a solution containing 100 ng of purified human genomic DNA and
- 50 µl of the following reaction mixture containing: 0.2 mM of the four triphosphate deoxynucleotides [dNTP Set 100 mM Solutions (dATP, dCTP, dGTP, dTTP) Cat. No. 27-2035-01, Amersham - Pharmacia], 200 ng of each oligonucleotide, 0.5 U of Taq DNA Polymerase and 10µl of reaction buffer 10x (Cat. No. 1-146-165 Boehringer Mannheim).

The sequences of the used oligonucleotides, respectively corresponding to the 5' and 3' ends of the gene of the human mature alpha 2b interferon, according to the sequence published in database under the number NM_000605, were as follows:

5'-ACGTCATATG**TGTGATCTGCCTCAAAC**-3',

and

5'-ACGTGGATCC**TCATTCCTTACTTCTTAA**-3'

that respectively introduce a NdeI site and a BamHI site (underlined) for the subsequent cloning of the vector.

The PCR amplification of the gene of the human alpha 2b interferon is carried out with this mixture by means of 35 cycles of amplification under the following conditions: one minute at 94°C, one minute at 56°C, one minute at 72°C.

The amplified DNA corresponds, as expected, to a fragment made of 518 nucleotides, codifying for the mature alpha 2b interferon (165 aa), with the addition of the sites of restriction NdeI and BamHI, necessary for its subsequent cloning in the expression vector.

The so amplified DNA fragment was:

- purified by means of suitable microcolumns (Jet Quick, PCR Purification Spin Column Kit 50 Cat. No. 410050, Genomed, Germany), according to the suggestions of the manufacturer;
- digested with the restriction enzymes NdeI and BamHI (Cat. No. 111-L and 136-L, New England Biolabs) according to the suggestions of the manufacturer;
- purified through a 1% agarose gel by using suitable microcolumns (Jet Quick, Gel Extraction Spin Kit 50, Cat. No. 420050, Genomed), according to the suggestions of the manufacturer;
- cloned in the pET9 plasmid (Cat. No. 69431-3, Novagen) predigested with the above mentioned enzymes and purified from agarose gel in a manner similar to that used for the fragment of amplified DNA.

The pET9 plasmid has been bound to the fragment purified and digested with the same enzymes, by using the T4-DNA ligase (T4 DNA Ligase Cat. No. 202-L, New England Biolabs, USA), according to the suggestions of the manufacturer.

The vector contains a gene for the resistance of the antibiotic kanamycin for the selection of the cells containing the plasmid. In fact the resistance to the kanamycin is preferable with respect to the resistance to the ampicillin more commonly used in laboratory for the expression of recombinant proteins. The kanamycin in fact does not decompose like the ampicillin, a thing that can cause the consequent loss of resistance of the strain, and according to the GMP (Good Manufacturing Practice) standards its use is preferable.

Competent cells DH5α (Max Efficiency DH5α Competent Cells Cat. No. 18258-012, Life Technology) were transformed with the product of the ligation according to the suggestions of the manufacturer. The transformed bacteria were seeded in LB-Agar plates containing 50 μg/ml of kanamycin. 18 Strains resistant to the kanamycin among the obtained strains were analyzed, growing them in 10 ml of LB medium containing 50 μg/ml of kanamycin until an $0D_{600}$ equal to 1.5.

The plasmidic DNA of these strains has been purified by using a commercial kit (Jet Quick Plasmid Miniprep Spin Quick Kit, Cat. No. 400250, Genomed). The presence of the gene of the alpha interferon in the pET9 plasmid has been verified by digestion with the restriction enzymes NdeI-BamHI and analysis on agarose gel as previously described.

Five clones resulted to be positive to the restriction analysis were sequenced in order to verify the absence of mutations due to the amplification process, by using an automatic sequentatior (CEQ 2000, Beckman - Coulter) with the following synthetic oligonucleotides:

5'-TAATACGACTCACTATA-3' T7 promoter

5'-GCTAGTTATTGCTCAGCGG-3' T7 terminator

The nucleotidic sequence of the isolated clones was confirmed to codify for the expression of the protein corresponding to the mature human alpha 2 interferon, as known in the literature and published in database under the number NM_000605.

Example 2

Optimization of the nucleotidic sequence codifying for the alpha 2b IFN for the expression in E. coli

The sequence of the human alpha 2b IFN was modified according to the preferential genetic code of the bacteria, in order to optimize its expression in E. coli.

The modifications in the nucleotidic sequence of the human IFN according to the "codon usage" of E. Coli were carried out according to the suggestions of J. Michael Cherry by using the program GCG "Codon Frequency" (ftp://ftp.es.embnet.org/pub/databases/codonusage/eco.cod).

The procedure comprises the reamplification by means of PCR of the fragment already isolated from human cells with the oligonucleotide:

**a)** which pairs with the 5' of the IFN sequence (seq. IDN2):

5'-agct<u>CATATG</u>TGTGATCTGCC**<u>G</u>**CAAACCCACAGCCTGGGTAGC**<u>C</u>**G**<u>TC</u>**G**<u>T</u>**-3'

and which introduces a Nde I site (underlined) and the substitutions shown in bold face and underlined (in italic the starting ATG codon) and

**b)** which pairs with the 3' of the IFN sequence (seq. IDN3):

5'-acgt<u>GGATCC</u>TCATTCCTTACTTCTTAA-**3'**

Such primers cause the substitution of the nucleotides in positions 12, 34, 36, 37, 39 of the sequence codifying for the mature human IFN (numeration according to the numeration of seq. IDN1).

The actg sequences at the beginning of each primer were added in order to make easier the cut procedures with the enzymes of restriction of the amplified product and its subsequent insertion in the expression vector.

The conditions and the reagents used for the reamplification of the gene of the alpha 2b interferon are those described in example 1 with the difference that instead of the human DNA, in the reaction mixture of amplification 10 ng of DNA of the plasmid containing the alpha 2b interferon already amplified and purified according to the description of example 1 were used. The fragment modified according to the codon usage of E. coli was cloned in the vector of expression pET9 of 5.5 kilobases, derived from the pBR322 plasmid, with the gene of the alpha interferon under the transcriptional control of the T7 promoter derived from the homonymous bacteriophage. The vector includes a terminator of the transcription in order to secure the exact termination of the transcript of the gene of the human alpha 2 interferon and signals of termination of the translation for all the three possible frameworks of reading. The resulting vector containing the gene of the interferon was then used for the transformation in competent cells DH5$\alpha$ and its sequencing carried out according to the description of example 1.

The sequence codifying for the mature human alpha 2b IFN, optimized according to the codon usage of E. coli, is shown in the list of sequences as seq. IDN1.

The DNA sequence coming from 5 different clones is the same and includes the modifications introduced from the synthetic oligonucleotides MUTS/IFN in the original sequence of the gene of the human alpha 2b IFN. The modifications introduced are underlined in the sequence of the preceding figure. The change observed in the codon 6 of the gene in all the analyzed clones (ACC *vs* ACT), does not change the resulting aminoacid and therefore it is accepted.

The DNA of the analyzed clones was used to transform competent cells BL21 DE3 (Cat. No. 69450-4, Novagen) and to check the levels of expression. The strains obtained in the LB-agar plates containing 50 µg/ml of kanamycin have been grown in liquid LB medium containing 50 µg/ml of kanamycin for a period of time of 15 h at 37°C in a thermostated stirrer at 250 rpm.

The strain of Escherichia coli used for the expression of the alpha 2 interferon contains in its chromosome a copy of the gene of the RNA polymerase coming from the T7 bacteriophage. This E. coli strain is lysogenic of the DE3 bacteriophage, a derived lambda phage which possesses the immune region of the 21 phage and brings a DNA fragment which codifies for the T7 RNA polymerase (Studier and Moffat, J. Mol. Biol., <u>189</u>, 113, 1986). The expression of the sequence codifying for the interferon is constitutive and has not to be induced with IPTG when the cells are grown in a type LB or TB medium at 37°C.

A part made of 100 µl of each of these cultures was analyzed in a SDS-PAGE gel containing 15% of polyacrylamide. The proteins were made visible in the gel through a coloration with the Coomassie Blue R-250 colorant.

A stripe having apparent molecular weight equal to 18.4 kDa, corresponding to the theoretical molecular weight of the interferon and having the same electrophoretic mobility as the International Standard of the alpha 2 interferon (Alpha 2b interferon CRS, cat. N° 320301, lot 2, and Alpha 2a interferon CRS, cat. N° 320300, lot 1, European Pharmacopoeia) was observed in all the 5 analyzed clones after expression.

The identity of this stripe was confirmed also by the use of commercial specific antibodies anti-alpha 2b interferon (alpha IFN, F18 clone, Cat. No. MON 5067, Monosan), by means of Western-blot.

<u>Example 3</u>

<u>Production of alpha interferon in E. coli</u>

This example describes the expression in E. coli of a sequence of DNA codifying for the mature human alpha 2b interferon, as obtained in example 2. The used sequence is therefore partly synthetic and partly derived from genomic DNA: the gene for the interferon does not contain introns and therefore it is equal to the sequence of the cDNA; synthetically, i.e. through amplification with synthetic primers, an initial methionine contained in the Ndel site was added, introduced in order to permit the synthesis of the mature protein, lacking the signal peptide and the part corresponding to the proteic precursor.

A part of competent cells of the BL21 DE3 strain of E. coli (Cat. No. 69451-4, Novagen) was transformed with 5 ng of the pET9-IFN-alpha 2b plasmid (see example 2) by using LB-agar plates containing kanamycin (50 μg/ml). The result of the transformation has been checked on the basis of the marker for the resistance to the kanamycin. The bacteria were grown in 1 l of LB medium containing kanamycin (50 μg/ml) at 37°C until a $OD_{600}$ equal to 2.

The level of expression of the interferon in the transformed bacteria, without any need of addition of IPGT inductor, was evaluated in an amount equal to about 10-15% of the total of the bacterial proteins, according to what observed in SDS-polyacrylamide gel coloured with Coomassie Blu R 250 (see figure 1, lane 1).

The cells were concentrated by centrifuging at 2,600 rpm in a Sorvall-GSA rotor. The pellet of bacteria was then suspended again in 50 ml of water, corresponding to no more than 15g wet weight of bacteria/100 ml and sonicated with a point of 9 mm (Soniprep-150, MSE, UK), at a power of 20μ, 5 cycles each of 60 seconds.

The sonicated material was recovered by centrifugation using a GSA-Sorvall rotor at 22,000 rpm for 30 minutes at 4°C. The pellet was suspended again in 50 ml of water and centrifuged as above.

The so obtained pellet of inclusion bodies was dissolved in 30 ml of a solution containing 6M Guanidine-HCl, 100 mM Tris HCl pH 8, 2mM EDTA, with a proteic concentration not higher than 10 mg/ml. The solution was stirred for 30 minutes at room temperature and then it was centrifuged in order to eliminate the possible non-resuspended particulate at 22,000 rpm for 30 minutes at 4°C.

The supernatant obtained after the centrifugation was diluted in about 2 litres of a buffer made of 0.5 M Arginine-HCl, 100 mM Tris HCl pH 8, 0.1 mM EDTA, 1 mM Cysteine, 0.05 mM Cystine, balancing the pH at 8 with a 0.1 M solution of NaOH until a proteic concentration equal to 200 μg/ml.

The renaturation mixture was submitted to a slow stirring (200 rpm on a magnetic stirrer) at 22°C for 24-36 hours.

The renaturation mixture was then dia-filtered against a buffer made of 20 mM Tris-HCl pH8 until a dilution value equal to 1:1250, by using an Amicon S1Y10 spiral cartridge, molecular cut 10000 d, 009 m2 Cat. N° 540630, with a CH2PRS spiral concentrator (cat. N° 54131, Amicon - Millipore). A dialysis or a de-salting by means of gel filtration on G-25 resin was carried out in some experiments instead of the dia-filtration or in addition to it.

The dia-filtrate was then brought to pH 5.5 with glacial acetic acid, by eliminating the precipitate through filtration (0.45 μm) and charged on a chromatographic column IEC-SP2825 (Shodex, Waters), which uses a matrix made of polyhydroxymethacrylate with sulphopropyl functional group, previously balanced with 50 mM sodium acetate, pH 5.5 at a flow equal to 8.0 ml/min. After charging the sample, the column was washed with a volume corresponding to 3 volumes of the column (where the volume of the column is equal to 154 ml) by using the same acetate buffer. The elution of the native alpha IFN, corresponding to the main peak c) of figure 2, was carried out by using a linear saline gradient 10%-40% of 0.5M NaCl in 50 mM pH 5.5 Na acetate buffer in 5 column volumes. The lower peaks a) and b) resolved from the chromatography, and corresponding to non-native forms of the protein, are shown in figure 2. The conductivity of the elution solution was checked and was found corresponding to 14 milliSiemens/cm.

The eluate (alpha 2 interferon under native conformation) resulted to have a concentration equal to 0.5 mg/ml. This concentrated solution was then sterilized by filtration on 0.22 micron filters and used for the quality analyses.

A column containing the Source 15S resin, a polystyrene matrix cross-linked with divinylbenzene (Pharmacia cat. N. 17-0944-01), having the methylsulfonate as functional group, previously balanced with a buffer made of 50 mM sodium acetate pH 5.5, was used in a series of other experiments obtaining yields fully comparable.

The analyses of the product eluted from the cationic exchange chromatography, carried out in accordance with the instructions of the European Pharmacopoeia, are reported in examples 4 and 5.

In tables 1-3 the yields of the renaturation and purification process as a whole are quoted, obtained starting from three different conditions of renaturation of the inclusion bodies.

Table 1:

| **yields of the renaturation and purification process in RB1 buffer.** | | | |
|---|---|---|---|
| RB1 | **Total protein (1)** | **IFN 2b mg (2)** | **% IFN vs total protein (3)** |
| RB t0 | 300 | 100 | 33% |
| RB t40 | 240 | 180 | 60% |

Table 1: (continued)

| **yields of the renaturation and purification process in RB1 buffer.** | | | |
|---|---|---|---|
| RB1 | **Total protein (1)** | **IFN 2b mg (2)** | **% IFN vs total protein (3)** |
| pH change | 190 | 140 | 47% |
| dia-filtration | 120 | 79 | 26% |
| chromatography | 70 | 69 | 23% |

The values in the table correspond to the average of 3 repetitions carried out on three different lots of renaturation under the optimum conditions of renaturation of the RB1 buffer (Tris EDTA, Arginine, cysteine/cystine).

*(1) Total protein: measured with the Bradford's method.*
*(2) interferon amounts: calculated with the percent areas collected in HPLC, [RP-column C18 Zorbax (0.25* 4.6)], on the basis of the retention time of the native interferon.*
*(3) IFN% versus total protein: ratio between the amount of interferon, in every phase of its purification, and the initial amount of protein.*

In tables 2 and 3 the yields of the different phases of the renaturation and purification process are reported, starting from different conditions of renaturation: in table 2 those corresponding to the renaturation in RB2 buffer (Tris 100 mM, pH8, EDTA 2mM, Arginine 0.5M), but in absence of the redox pair, or also in absence of arginine (results in table 3) corresponding to the renaturation in RB3 buffer: Tris 100 mM, pH8, EDTA 2mM.

Table 2:

| **yields of the refolding and purification process in RB2 buffer** (Tris, EDTA, Arginine). | | | |
|---|---|---|---|
| RB2 | Total protein (1) | IFN 2b mg (2) | % IFN vs total protein (3) |
| RB t0 | 300 | 45 | 15% |
| RB t40 | 250 | 110 | 37% |
| pH change | 230 | 101 | 34% |
| dia-filtration | 73 | 34 | 18% |
| chromatography | 25 | 24 | 8% |

The values in the table correspond to the average of 3 replications carried out on three different batches of renaturation in absence of the redox pair. (1), (2) and (3) like in table 1.

Table 3:

| **yields of the refolding and purification process in RB3 buffer** (Tris, EDTA). | | | |
|---|---|---|---|
| RB3 | Total protein (1) | IFN 2b mg (2) | % IFN vs total protein (3) |
| RB t0 | 300 | 13 | 4% |
| RB t40 | 240 | 50 | 17% |
| pH change | 190 | 41 | 14% |
| dia-filtration | 120 | 34 | 11% |
| chromatography | 70 | 20 | 7% |

The values in the table correspond to the average of 3 replications carried out on three different batches of renaturation in renaturation buffer lacking both the redox pair and the arginine. (1), (2) and (3) like in table 1.

The data shown in tables 1-3 demonstrate that under the renaturation and purification conditions according to the present invention, corresponding to the conditions of renaturation of the RB1 buffer, the end yield of product increases in a remarkable manner (more than 3 times) with respect to the renaturation conditions of the RB3, or also RB2, buffer. The remarkable difference refers not only to the yields, but also to the biological activity and to the purity of the product, as described in example 4.

## EXAMPLE 4

### Identity and purity analysis of the recombinant alpha 2 IFN according to the invention, carried out in accordance with the guidelines of the European Pharmacopoeia

The guidelines of the "European Pharmacopoeia (Alpha 2 interferon concentrated solution, 1110 (1031-1035), 1997) provide for the following analytic procedures: SDS-PAGE; isoelectrofocalization; peptidic map after tryptic digestion; reverse phase HPLC (see Figures 2 to 8), determination of the biological power, determination of the endotoxins content. Further analyses were carried out in addition.

The results obtained in the different tests show that the alpha 2 IFN produced according to the invention is qualitatively comparable, and in some tests also superior, to that used as reference standard obtained from the "Council of Europe", European Pharmacopoeia Commission, Biological Standardisation, Strasbourg, Alpha 2b interferon CRS (Batch No. 2) cat. N° 1320301 and Alpha 2a interferon CRS (Batch No. 1), cat. N° 1320300.

Therefore standard interferon, wherein not explicitly mentioned, means either standard preparation, while the monography on the alpha interferon published on "European Pharmacopoeia" Alpha 2 interferon concentrated solution, 1110 (1031-1035), 1997 means guidelines of the European Pharmacopoeia.

The electrophoresis on SDS-PAGE under reducing conditions (coloured with Coomassie) of the different phases of the preparation process of IFN is shown in figure 1. The advantage of working with the inclusion bodies, according to the preferred fulfilment of the invention, is evident: in fact, the content of recombinant IFN, even if under non-native configuration, succeeds in forming about 90% of the total proteins, as it can be seen in lane 4 of figure 1. This purity degree is obtained in only two steps: the breaking of the bacterial cells by sonication in water and a subsequent centrifugation, in absence of detergents or of particular reagents.

The chromatographic profile of the alpha 2 interferon in the ionic exchange chromatography (Shodex 2825 column) is shown in Figure 2. The resolution of the peak c) corresponding to the native form of the alpha IFN has to be noted compared with both peak a) and peak b), instead corresponding to non-native configurations of alpha 2 IFN. Such resolution is obtained through a sole chromatographic step.

The purity of the material eluted and corresponding to the peak c) of figures 2 was analyzed by electrophoresis on SDS-PAGE under non-reducing (figure 3a) and reducing (figure 3b) conditions, developed with silver coloration, in accordance with what established in the guidelines of the European Pharmacopoeia, already cited Monography on the interferon, in order to show the possible presence of impurities having a molecular weight different from those of the native alpha 2 IFN, the dimers of alpha IFN among them. Two dilutions of the preparation of interferon according to the invention and five dilutions of standard IFN from the European Pharmacopoeia (STD EP), as described in the corresponding legend, were charged in the electrophoresis shown in figures 3a and 3b.

The purity degree with reference to the content of dimers of alpha IFN (corresponding to non-native forms of the protein) can be shown under non-reducing conditions (figure 3a). The intensity of the stripe having a molecular weight higher than the molecular weight of the main stripe, present in lane 3 (wherein 25 µg of the IFN preparation according to the invention were charged) is comparable to the penultimate dilution of the standard (250 ng) which corresponds to 1% of the amount of the sample according to the invention charged in lane 3). Therefore the purity of the IFN preparation with respect to the content of dimers and calculated on the basis of the guidelines of the European Pharmacopoeia, (Alpha 2 interferon concentrated solution, 1110 (1031-1035), 1997) is at least 99%.

Stripes having molecular weight lower than that of the main stripe, made of monomeric alpha IFN of 19.3 kDa, and in case attributable to degradation products of IFN, are not noticeable in figure 3b) corresponding to the electrophoresis under reducing conditions.

The sensitivity of the method permits to see the last dilution of the IFN STD-EP preparation, corresponding to 50 ng, and equal to 0.2% of the stripe of the IFN according to the invention, 25µg, charged in lane 3.

Therefore the purity degree of the IFN preparation according to the invention versus impurities having lower molecular weight results to be equal to 99.8% according to this analytical method.

The Western-Blot of the preparation of purified interferon developed with anti-E. coli antibody for the detection of possible bacterial contaminant shows in figure 4a) that in the preparation of IFN according to the invention detectable bacterial contaminants are not present and in figure 4b) that the produced interferon is recognized from an anti-alpha IFN antibody.

The gel of isoelectrofocalization of the alpha 2b interferon according to the invention and to the reference standard carried out in agreement with the European Pharmacopoeia is shown in figure 5a). The isoelectric point, calculated by placing in the diagram (figure 5b) the migration distances of the reference standards and interpolating the corresponding value to the reference standard, results to be equal to 5.92, identical to that of the standard. The gel has run in accordance with the indications of the European Pharmacopoeia for the alpha IFN and using the following reagents: Electrophoresis equipment: Multiphor II Electrophoresis Unit, Cat. No. 18-1018-06, Pharmacia. Power supply: EPS 3500, Cat. No. 19-3500-00, Pharmacia. Refrigerated Water Bath: Multi Temp III Thermostatic Circulator: Cat. No. 18-1102-78, Pharmacia. pl. Markers: Broad pl-Kit pH 3.5-9.3, Cat. No. 17-0471-01, Pharmacia. IEF-Gel: Ampholine PAGplate IEF pH 3.5-9.5, Cat. No. 80-1031-60, Pharmacia. IFN-Standard: alpha 2b interferon, Cat. No. I0330301. EDQM-European Pharmacopoeia. Anodic solution: phosphoric acid 1M Cat. No. 100563, Merck. Catodic solution: 1M NaOH, Cat. No. 109956.0001, Merck. Applicators: Sample Application pieces, Cat. No. 80-1129-46, Pharmacia. Glacial Acetic acid: Cat. No. 10015N, AnalaR, BDH Ethanol: Cat. No. 10107, AnalaR, BDH. Trichloroacetic acid: Cat. No. 91234, Fluka. Sulphosalicylic acid: 2-Hidroxy-5-sulfobenzoic acid, Cat. No. 86193, Fluka.

The isoelectric point, calculated by interpolation on the diagram shown in figure 5b, of the interferon obtained according to the invention under the three renaturation conditions (RB1, 2 and 3 buffers) and of the EP standard is shown in table 4. The isoelectric point calculated for the three samples and for the standard results to be equal to 5.92.

Table 4

| ISOELECTRIC POINT CALCULATED FROM GEL | |
|---|---|
| EP std | 5.92 |
| RB1 | 5.92 |
| RB2 | 5.92 |
| RB3 | 5.92 |

The reverse phase chromatography of the alpha 2b interferon and of the standard alpha 2b interferon is shown in figure 6. The area corresponding to the peak of the alpha 2b IFN obtained according to the present invention corresponds to the 98.81% of the total area of the chromatogram, with a retention time equal to 54.56 minutes. The area of the peak corresponding to the EP standard alpha 2 IFN results to be equal to 97.45% of the total area (lower than that of the preparation according to the invention), with a very similar retention time (54.66 min.).

The tryptic map of the alpha 2b interferon obtained according to the invention, (panel a) upper portion of the figure) and of the preparation of standard IFN (panel b) under portion of the figure) is shown in figure 7. The map was obtained according to the requirements from the European Pharmacopoeia "Monography on interferon" by using the following reagents: Materials and reagents: HPLC equipment: AKTA purifier 10, Cat. No. 18-1400-00, Pharmacia. Column: Sephasyl Peptide C 18, 5um, 4.6/100 mm, Cat. No. 17-6000-26, Pharmacia. Trypsin (Sequencing grade, Cat. No. 8658. Sigma). Potassium dihydrogen phosphate: Cat. No. 60219, Fluka. Sodium hydroxide: Cat. No. 71690, Fluka. Guanidine hydrochloride: Cat. No. 50935, Fluka. Dithiothreitol: Cat. No. D-5545, Sigma. Acetonitrile: Hipersolve, Cat. No. 152516Q, BDH. Trifluoroacetic acid: Cat. No. 108262, Merck. Reference interferon: Alpha 2b interferon CRS (Batch No. 1, Council of Europe, European Pharmacopoeia Commission, Biological Standardisation).

It is possible to observe that the tryptic map is qualitatively similar to that of the reference standard in the under portion, showing that the peptides produced by the digestion with trypsin both in the product and in the EP standard are qualitatively and quantitatively the same, and that therefore there are no alterations post-translational or related to the purification process in the end product, obtained according to the invention, with respect to the control standard.

The IFN produced with the process of the invention was submitted to Mass Spectroscopy by means of Ion-Spray-PE SCIEX, AP1150EX, Perkin Elmer (figure 8), in addition to the requirements from the European Pharmacopoeia.

The values of molecular mass obtained by means of Ion Spray that further confirm the structural identity of the alpha 2b IFN according to the invention with respect to the international standard of alpha 2 IFN are shown in figure 8. The difference of molecular mass observed between the two products (19262.79 versus 19262.82, lower than 0.03 mass units) falls within the interval of experimental error of the instrument. This datum confirms that the alpha 2b IFN produced according to the invention corresponds to the mature IFN and is absolutely equivalent to the standard of the European Pharmacopoeia at molecular level.

**EXAMPLE 5**

**Determination of the biological power and of the endotoxins content of the alpha interferon prepared according to the process of the invention.**

*Determination of the biological power.*

The determination of the biological power of the alpha IFN produced was checked, in agreement with the "European Pharmacopoeia", on MDBK cells (NBL-1, Madin Darby Bovine Kidney cells) ATCC Cat. N° CCL-22, infected with the Vescicular Stomatitis Virus, according to the procedure described in Rubinstein S. et al., J. Virol., 37; 755-758, 1981 and in Meager A. 129-147, 1987 in "Lymphokines and Interferons", Clemens M.J., Morris A.G. and Gearing A. J.H., editors, IRL Press, USA. Shortly, the test has been carried out by using microplates with 96 wells containing $3x10^4$ cells for each well, incubated at 37°C 5% $CO_2$ for 18 hours. The cells were pre-treated with the samples of alpha IFN according to the invention, obtained under the three different conditions of renaturation, at different dilutions, and with the standard of the European Pharmacopoeia, for 22 hours before the infection with the VSV virus ($5x10^4$ pfu/ml, amount necessary to lyse cells not protected from the alpha IFN within 24 hours from the infection). The quantification of the residual cytopathogenic effect was carried out by means of coloration with neutral red and spectrophotometric reading with a plates reader Spectracount Cat. BS10001, Packard Instrument.

The statistical treatment of the data was fulfilled as provided for from the European Pharmacopoeia and described in "Statistical analysis of results of biological assays and tests" European Pharmacopoeia 1997, 299-336. The data are reported in table 5 from which it results that the estimated power is never lower than 80% (89%) nor higher than 125% (122%) of the measured power in the preparation of the alpha 2 interferon according to the invention. The fiduciary limits of measurement at p = 0.95 are comprised between 64% and 156% of the measured power and therefore they are comprised within the limits provided for from the European Pharmacopoeia. The biological activity of the sample prepared according to the invention, corresponding to RB1 sample, is comprised between 2.85 and $3.07x10^8$ IU/mg of protein versus a biological activity of the reference standard equal to $2.4x10^8$ IU/mg of protein. The reference standard with the purpose of the evaluation of the biological activity, was made of "1$^{st}$ International Standard - Recombinant human interferon alpha 2b (82/576) NIBSC (National Institute for biological standards and control, Blanche Lane, South/mimms, Potters Bar, Eng. 3QG, UK) and was obtained by resuspending an aliquot corresponding to a 17,000 IU in a suitable volume.

It is moreover possible to see from the data of table 5 that the renaturation conditions in the RB1 renaturation solution (presence of a redox pair like the cysteine/cystine pair with respect to the renaturation solution RB2 and furthermore of a labilizing agent, like the arginine, with respect to the RB3 solution) allows not only a higher yield of end product, as illustrated in tables from 1 to 3, but also a better quality of the interferon produced. Therefore the RB1 renaturation buffer is the optimum renaturation buffer.

**Table 5: determination of the biological activity of the recombinant interferon according to the present invention.**

| Renaturation buffer | Proteic concentration (Lowry) mg/ml | Estimated biological activity (IU/mg) | Measured biological activity (IU/mg) | Measured/estimated biological activity (IU/mg) | Fiduciary Limit (P 0.95) lower | Fiduciary Limit (P 0.95) higher |
|---|---|---|---|---|---|---|
| RB1 | 0.5 mg/ml | $3 \times 10^8$ | $3.07 \times 10^8$ | 103% | 74% | 142% |
| | 0.48 " | | $3.47 \times 10^8$ | 116% | 82% | 136% |
| | 0.53 | | $2.85 \times 10^8$ | 95% | 69% | 129% |
| RB2 | 0.27 mg/ml | $1.5 \times 10^8$ | $1.83 \times 10^8$ | 122% | 69% | 133% |
| | 0.28 " | | $1.47 \times 10^8$ | 98% | 91% | 144% |
| | 0.29 | | $1.63 \times 10^8$ | 109% | 85% | 145% |
| RB3 | 0.25 mg/ml | $2.5 \times 10^8$ | $2.23 \times 10^8$ | 89% | 74% | 129% |
| | 0.25 " | | $2.88 \times 10^8$ | 115% | 83% | 141% |
| | 0.26 | | $2.59 \times 10^8$ | 104% | 79% | 130% |
| Confidence limits according to the European Pharmacopoeia | | | | 80%<x>125% | 64%<x<156% | |

Determination of the endotoxins content.

The determination of the endotoxins content was checked on the three IFN preparations (RB1, RB2 and RB3 according to what described in example 3) by means of the test based on LAL (Limulus Amoebocyte Lysate, "Guideline on Validation of Limulus Amoebocyte Lysate for Human and Animal parenteral Drugs." Biological Products and Medical Devices, December, 1987) of the Endosafe kit (Charles River, Ltd) and following the manufacturer's instructions. The used reagents were: Gel clot reagent, sensitivity 0.125 EU/ml, (Charles River, Cat. No. AE040), Standard endotoxin CSE E. coli (055:B5) (Charles River, Cat. No. AE075), Apyrogenic water 50 ml, (Cat. No. AE089), Test-tubes made of borosilicate 13*100 mm (Cat. No. AE112).

The endotoxins content was comprised between 2.5 and 25 IU in the volume containing 1 mg of protein in all three production batches corresponding to the three different renaturation conditions, as it can be seen from the results shown in table 6. The measured value results therefore to be lower than the value of 100 IU in the volume containing 1 mg of protein provided for according to the European Pharmacopoeia for the alpha interferon of therapeutical degree.

TABLE 6

| **Endotoxins content in the preparation of purified interferon coming from the three renaturation mixtures RB1, RB2 and RB3 (RB1, 2 and 3 defined as in tables 1-3).** | | |
|---|---|---|
| **Renaturation buffer** | **Proteic concentration** | **Endotoxins concentration** |
| RB1 | 0.5 mg/ml | 2.5 IU/mg<[endotoxin]<25IU/mg |
| RB2 | 0.27 mg/ml | 4.6 IU/mg<[endotoxin]<23IU/mg |
| RB3 | 0.25 mg/ml | 5 IU/mg<[endotoxin]<25IU/mg |

The data presented in table 6 show that the purification process gives an endotoxins content lower to that established from the European Pharmacopoeia irrespectively of the type of renaturation solution used to get the alpha interferon under native conformation.

List of sequences

**Seq. IDN.1**

**Mature alpha 2b IFN sequence optimized according to the preferential use of the E. coli codons:**

| | | | |
|---|---|---|---|
| TGTGATCTGC | CGCAAACTCA | CAGCCTGGGT | AGCCGTCGTA |
| CCTTGATGCT | CCTGGCACAG | ATGAGGAGAA | TCTCTCTTTT |
| CTCCTGCTTG | AAGGACAGAC | ATGACTTTGG | ATTTCCCCAG |
| GAGGAGTTTG | GCAACCAGTT | CCAAAAGGCT | GAAACCATCC |
| CTGTCCTCCA | TGAGATGATC | CAGCAGATCT | TCAATCTCTT |
| CAGCACAAAG | GACTCATCTG | CTGCTTGGGA | TGAGACCCTC |
| CTAGACAAAT | TCTACACTGA | ACTCTACCAG | CAGCTGAATG |
| ACCTGGAAGC | CTGTGTGATA | CAGGGGGTGG | GGGTGACAGA |
| GACTCCCCTG | ATGAAGGAAGG | ACTCCATTCT | GGCTGTGAGG |
| AAATACTTCC | AAAGAATCAC | TCTCTATCTG | AAAGAGAAGA |
| AATACAGCCC | TTGTGCCTGG | GAGGTTGTCA | GAGCAGAAAT |
| CATGAGATCT | TTTTCTTTGT | CAACAAACTT | GCAAGAAAGT |
| TTAAGAAGTA | AGGAA**TGA** | | |

**Seq. IDN.2**

**Oligonucleotide used for the amplification of the 5' of the alpha 2 IFN modified according to the preferential use of the E. coli codons (modification of the 12, 34, 36, 37, 39 nucleotides numeration in accordance with seq. idn1)**

AgctCATATG TGTGATCTGC CGCAAACCCA CAGCCTGGGT AGCCGTCGT

**Seq. IDN. 3**

**Oligonucleotide used for the amplification of the 3' of the alpha 2 IFN.**

acgtGGATCCTCATTCCTTACTTCTTAA

## Claims

1. Process for the purification of alpha interferon under native conformation and of therapeutical degree from a denaturation mixture, which comprises the following steps:

   a) oxidizing renaturation;
   b) chromatography on a strong cationic exchanger and elution.

2. Process according to claim 1 wherein such alpha IFN is recombinant.

3. Process according to claim 2 wherein such recombinant alpha IFN is human.

4. Process according to claim 3 wherein such alpha IFN is alpha 2 IFN.

5. Process according to claims 1 to 4 wherein the oxidizing renaturation takes place by slow dilution of the denaturation mixture within a renaturing solution.

6. Process according to claim 5, wherein the end concentration of the alpha IFN after dilution is comprised between 50 and 300 μg/ml.

7. Process according to claim 5 wherein such renaturing solution is a buffer system having pH comprised between 7.5 and 8.5, preferably pH 8.0±0.1, comprising a labilizing agent, a redox pair and, optionally, a chelating compound.

8. Process according to claim 7 wherein the labilizing agent is L-arginine or salts and derivatives thereof.

9. Process according to claim 8 wherein the concentration of the L-arginine or salts and derivatives thereof is comprised between 0.3 and 0.6 M.

10. Process according to claim 9 wherein the concentration is comprised between 0.45 and 0.55 M.

11. Process according to claim 7 wherein the redox pair is selected from: glutathione under oxidized and reduced form, cystine and cysteine, cystamine and cysteamine, hydroxyethylsulfoxide and 2-mercaptoethanol.

12. Process according to claim 11 wherein the redox pair is L-cysteine and salts or derivatives thereof at a concentration comprised between 0.1 and 10 mM and L-cystine and salts or derivatives thereof at a concentration comprised between 0.005 and 0.5 mM.

13. Process according to claim 12 wherein the L-cysteine is at a concentration comprised between 0.5 and 2 mM and the L-cystine is at a concentration comprised between 0.03 and 0.07 mM.

14. Process according to claims 7 to 13 wherein the oxidizing renaturation takes place at a pH of the renaturation mixture comprised between 7.5 and 8.5, preferably pH 8.0±0.1 and at a temperature comprised between 5°C and 25°C.

15. Process according to claim 14 wherein the oxidizing renaturation takes place in a period comprised between 18 and 50 hours.

16. Process according to claims 1 to 15 **characterised in that** at least a step of de-salting of the renaturation mixture is comprised and optionally a step of concentration.

17. Process according to claim 16 wherein the de-salting is carried out before the step b) of chromatography.

18. Process according to claim 16 wherein the de-salting takes place according to one of the following methods: dialysis, ultra-filtration, gel filtration, dia-filtration.

19. Process according to claims 1 to 18 wherein the renaturation mixture, coming from the step a) of the process, is brought to a pH comprised between 5.3 and 5.7, preferably pH 5.5 ± 0.1, before the step b) of the process and then optionally centrifuged or filtered.

**20.** Process according to claims 1 to 18 wherein the chromatography of the step b) of the process takes place on a matrix in which the functional group is selected from sulfopropyl and methylsulfonate.

**21.** Process according to claim 20 wherein the chromatography takes place at a pH comprised between 5.3 and 5.7, preferably pH 5.5±0.1.

**22.** Process according to claim 21 wherein the chromatography takes place in acetate buffer.

**23.** Process according to claims 20 to 22 wherein the elution of the alpha IFN from the chromatographic column takes place by application of a linear saline gradient of a monovalent salt at a concentration comprised between 0.001 and 1 M.

**24.** Process according to claim 23 wherein such salt is sodium chloride and wherein such gradient goes from 0.001 to 0.5 M.

**25.** Process according to claims 20 to 22 wherein the elution of the alpha IFN takes place with an isocratic solution of an acetate buffer comprising a monovalent salt, preferably sodium chloride.

**26.** Process according to claims 22 to 25 wherein the acetate buffer is sodium acetate.

**27.** Process for the production of alpha interferon of therapeutical degree **characterised in that** it comprises the purification process claimed in claims 1 to 26.

**28.** Process according to claim 27 **characterised in that** it comprises the following preliminary steps:

- 1- intracellular expression of a sequence of DNA codifying for the alpha interferon in a prokaryotic organism or in a simple eukaryote, genetically modified;
- 2- recovery of alpha interferon under non-native form;
- 3- treatment of the alpha interferon with chaotropic agents.

**29.** Process according to claim 28 wherein such prokaryotic organism is selected from E. coli and B. subtilis.

**30.** Process according to claim 29 wherein such prokaryote is E. coli.

**31.** Process according to claim 28 wherein such eukaryotic organism is selected from S. cerevisiae, P. pastoris and K. lactis.

**32.** Process according to claims 28 to 30 wherein the recovery of the interferon under non-native form is obtained by centrifugation of the bacterial culture, lysis of the bacteria in an aqueous solution and centrifugation of the inclusion bodies.

**33.** Process according to claim 32 wherein the lysis of the bacteria is obtained by sonication.

**34.** Process for the preparation of human recombinant alpha 2 IFN essentially comprising the following steps:

i) expression of a sequence codifying for the human alpha 2 IFN in E.coli;
ii) lysis of the bacteria and recovery of the inclusion bodies in an aqueous solution;
iii) treatment of the inclusion bodies with a concentrated solution of a chaotropic agent at a concentration comprised between 5 and 7 M, buffered at a pH comprised between 7.5 and 8.5;
iv) dilution of the denaturation solution in a renaturation solution comprising L-arginine or salts or derivatives thereof at a concentration comprised between 0.45 and 0.55 M, the redox pair L-cysteine/cystine or salts or derivatives thereof, at a concentration respectively comprised between 0.5 and 2mM (L-cysteine or salts and derivatives thereof) and between 0.03 and 0.07mM (L-cystine or salts and derivatives thereof), and having after dilution an end proteic concentration comprised between 100 and 250 µg/ml and an end pH equal to 8.0±0.1;
v) chromatography in an acetate buffer at a pH comprised between 5.3 and 5.7, preferably pH 5.5 ± 0.1, on matrix with functional group with strong cationic exchange selected from sulfopropyl and methylsulfonate;
vi) elution in linear saline gradient from 0 to 500 mM of NaCl in acetate buffer at pH comprised between 5.3 and 5.7, preferably pH 5.5±0.1.

**35.** Process according to claim 34 wherein at item vi) the elution takes place isocratically with a saline solution having conductivity comprised between 5 and 7 milliSiemens/cm, preferably 6 milliSiemens/cm.

**36.** Process according to claims 34 and 35 wherein the sequence codifying for the alpha 2 IFN is the sequence identified on databank with the number NM_000605, optimized according to the use of the preferential codons of E. coli.

**37.** Process according to claim 36 wherein such sequence is the IDN1 sequence enclosed in the list of the sequences.

**38.** Process according to the claims 34 to 37 wherein such chaotropic agent is GuHCl at a concentration comprised between 5 and 7 M, preferably 6 M, and wherein the proteins have an end concentration comprised between 6 and 14 mg/ml.

**39.** Alpha interferon under native conformation and of therapeutical degree obtainable in accordance with the process claimed in claim 27.

**40.** Recombinant alpha 2 interferon under native conformation and of therapeutical degree obtainable in accordance with the process claimed in claims 34 to 38.

**41.** Alpha 2 interferon according to claim 40 **characterized in that** it has a purity degree higher than 99% by SDS-PAGE.

**42.** Alpha 2 interferon according to claims 40 and 41 **characterised in that** it has a biological activity not lower than 2.5 x $10^8$ IU/mg, preferably not lower than 3 x $10^8$ IU/mg.

**43.** Alpha 2 interferon according to claims 40 to 42 **characterised in that** it has a content of endotoxins lower than 25 IU/ml.

**44.** Alpha 2 interferon according to claims 40 to 43 for therapeutical use.

**45.** Use of the alpha 2 interferon according to claim 44 for the preparation of antiviral medicines.

**46.** Use of the alpha 2 interferon according to claim 44 for the preparation of antitumoral medicines.

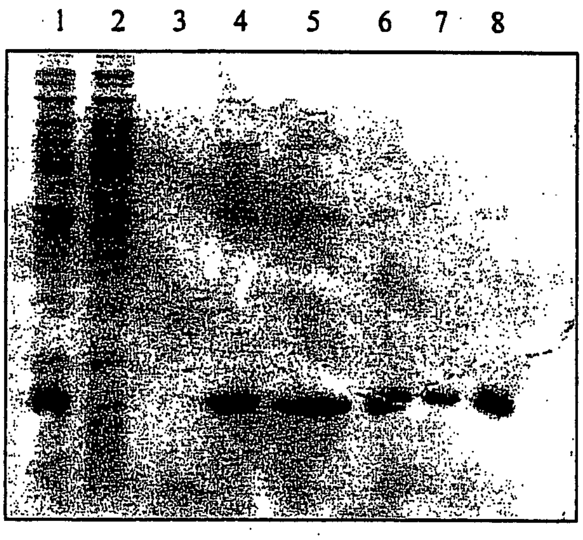


FIGURE 1

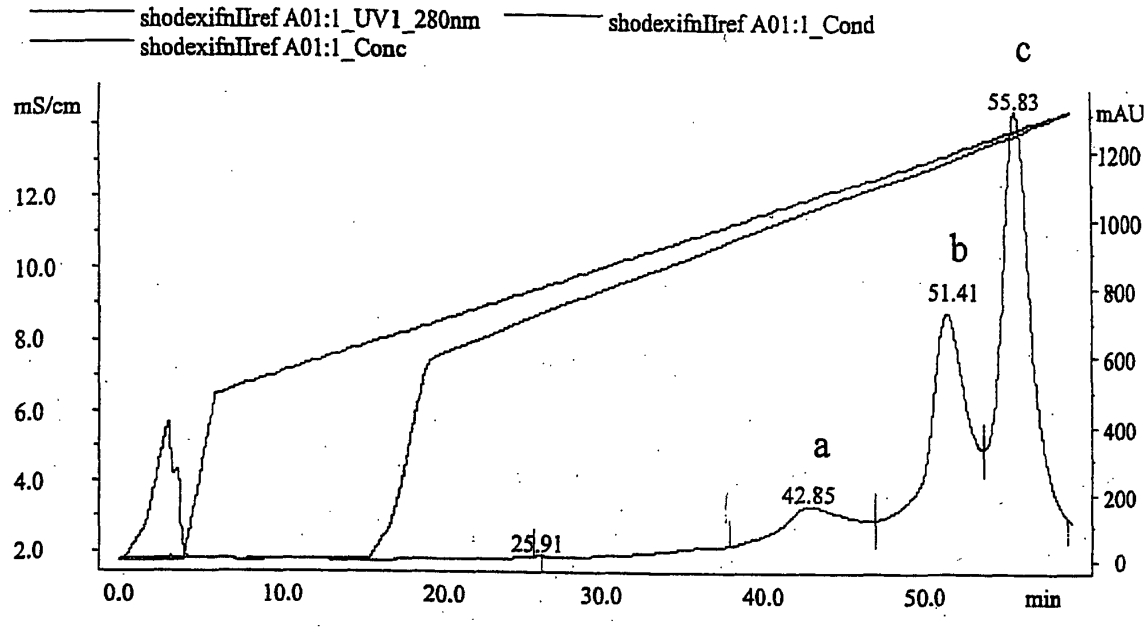
shodexifnIIref A01:1_UV1_280nm
shodexifnIIref A01:1_Cond
shodexifnIIref A01:1_Conc
mS/cm
mAU
c
55.83
b
51.41
a
42.85
25.91
12.0
10.0
8.0
6.0
4.0
2.0
1200
1000
800
600
400
200
0
0.0
10.0
20.0
30.0
40.0
50.0
min

a)

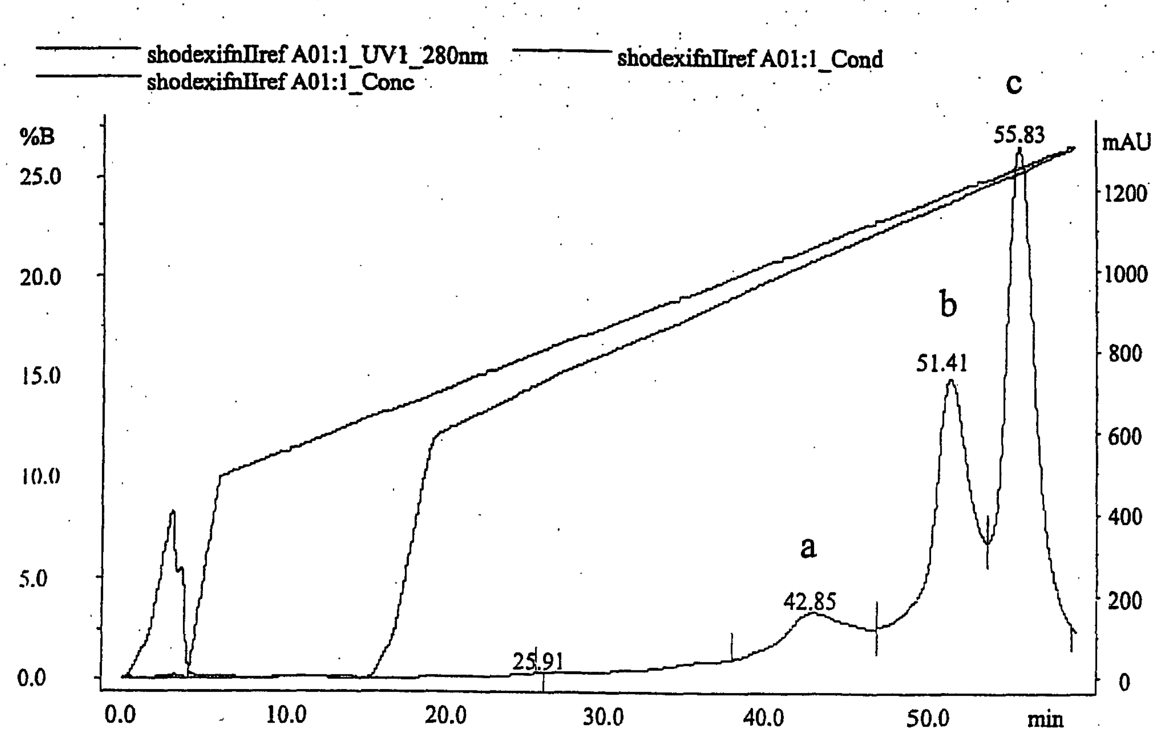
shodexifnIIref A01:1_UV1_280nm
shodexifnIIref A01:1_Cond
shodexifnIIref A01:1_Conc
%B
mAU
c
55.83
b
51.41
a
42.85
25.91
25.0
20.0
15.0
10.0
5.0
0.0
1200
1000
800
600
400
200
0
0.0
10.0
20.0
30.0
40.0
50.0
min

b)

FIGURE 2

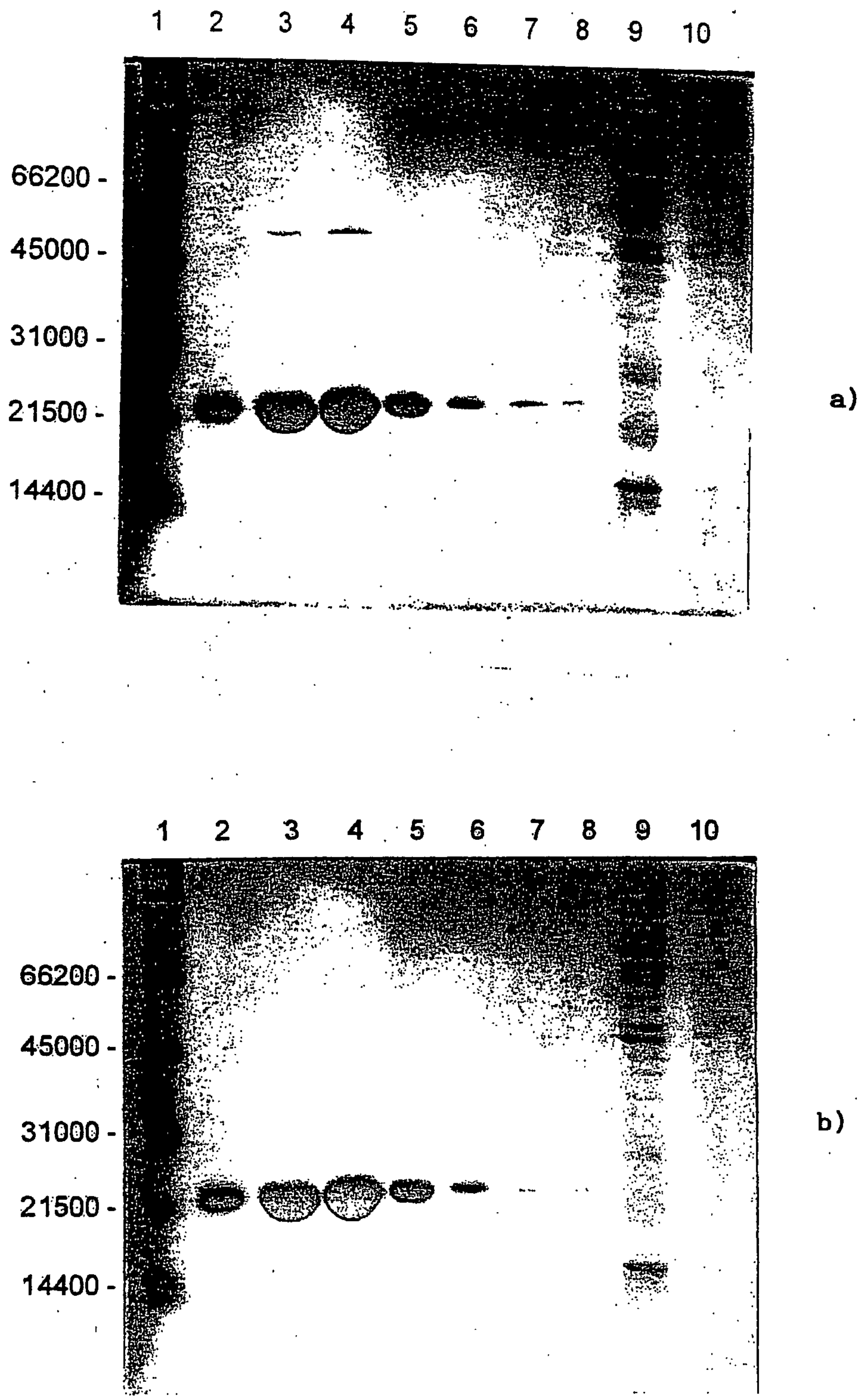
1 2 3 4 5 6 7 8 9 10
66200 -
45000 -
31000 -
21500 -
14400 -
a)
1 2 3 4 5 6 7 8 9 10
66200 -
45000 -
31000 -
21500 -
14400 -
b)

FIGURE 3

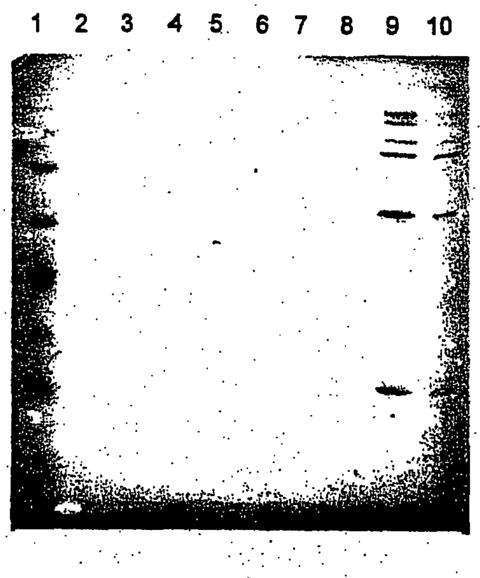


FIGURE 4a

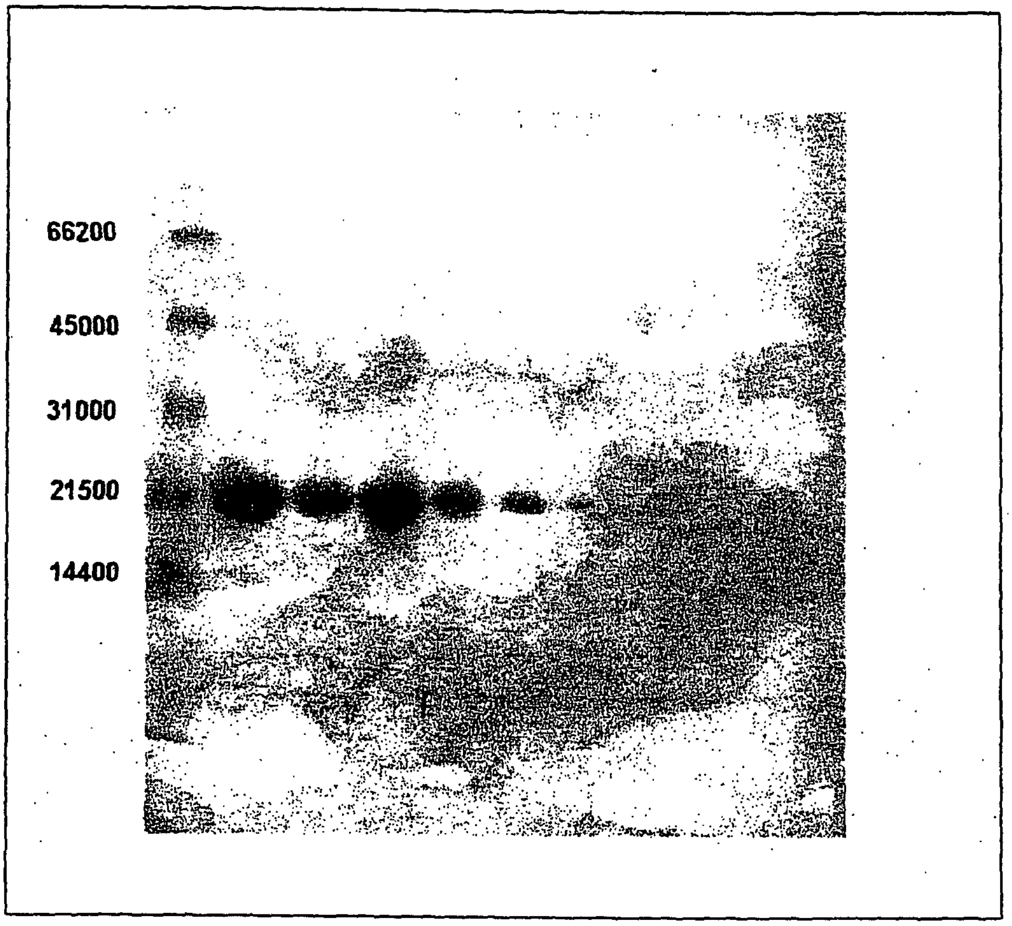


FIGURE 4b

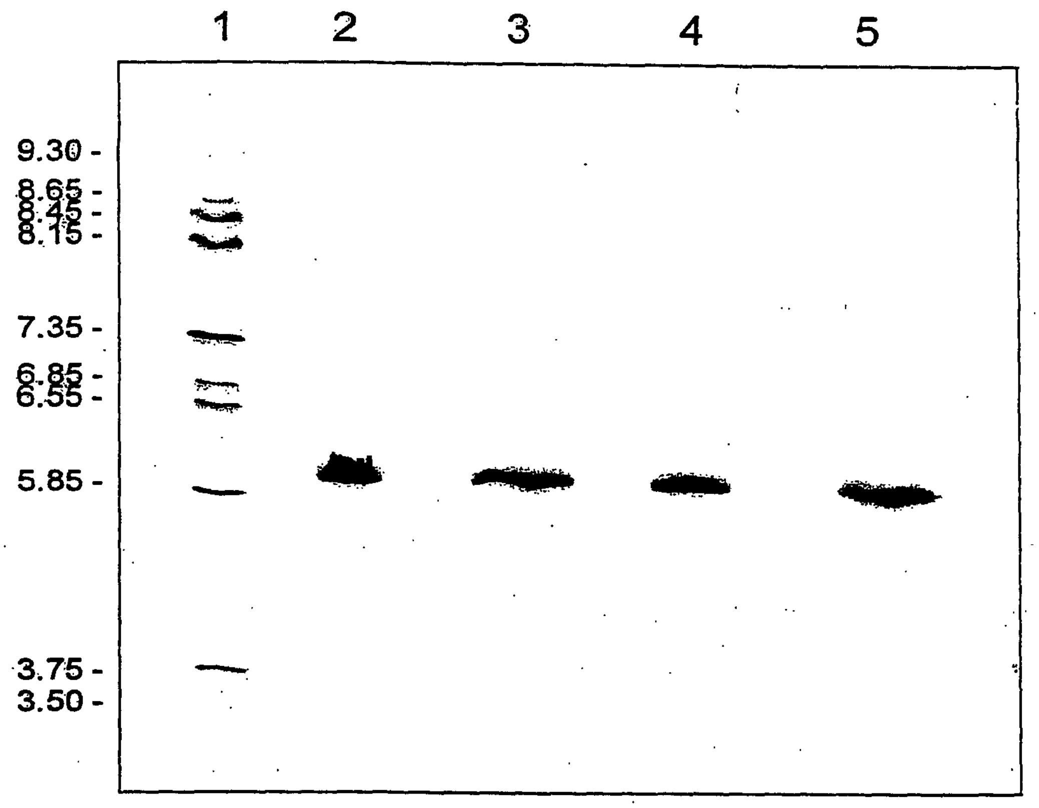
1
2
3
4
5
9.30
8.65
8.45
8.15
7.35
6.85
6.55
5.85
3.75
3.50

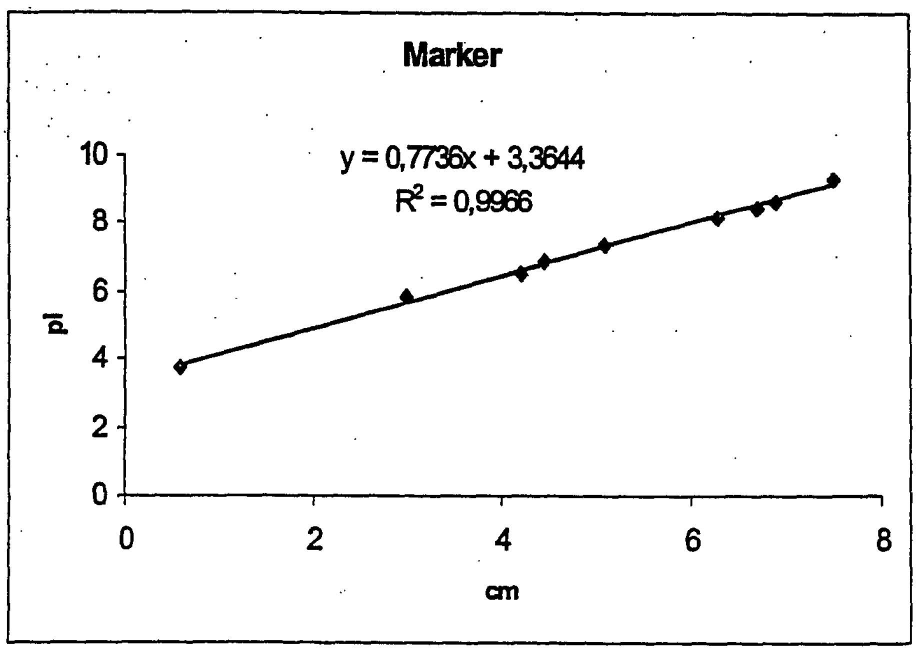
Marker
y = 0,7736x + 3,3644
R2 = 0,9966
pI
cm
0
2
4
6
8
10

FIGURE 5

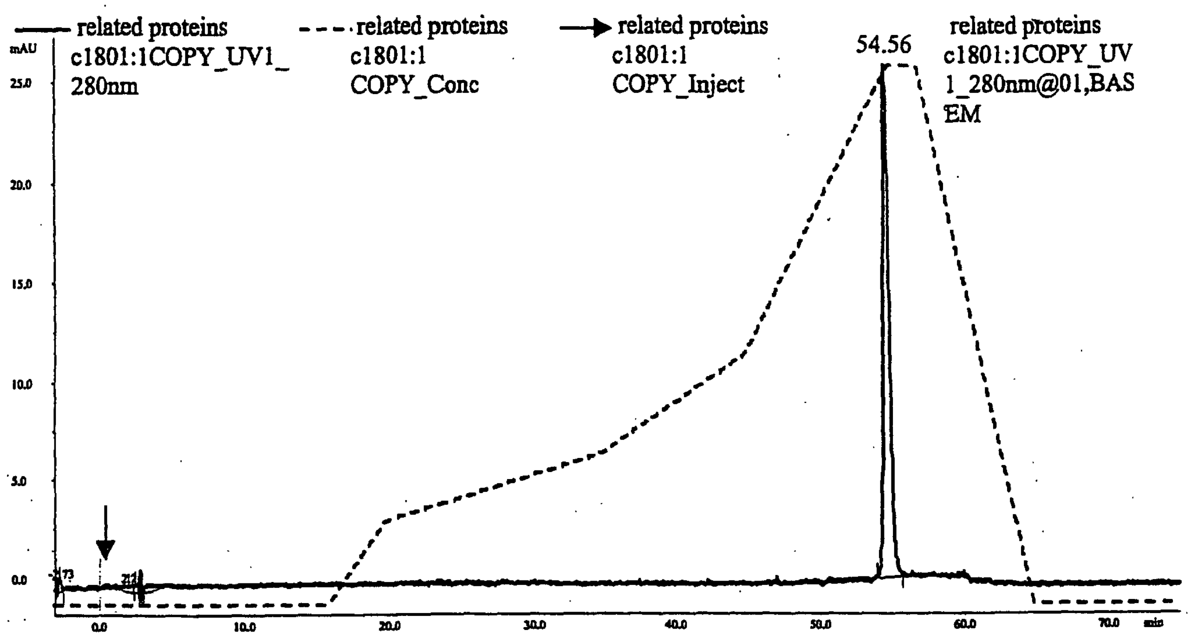


FIGURE 6a

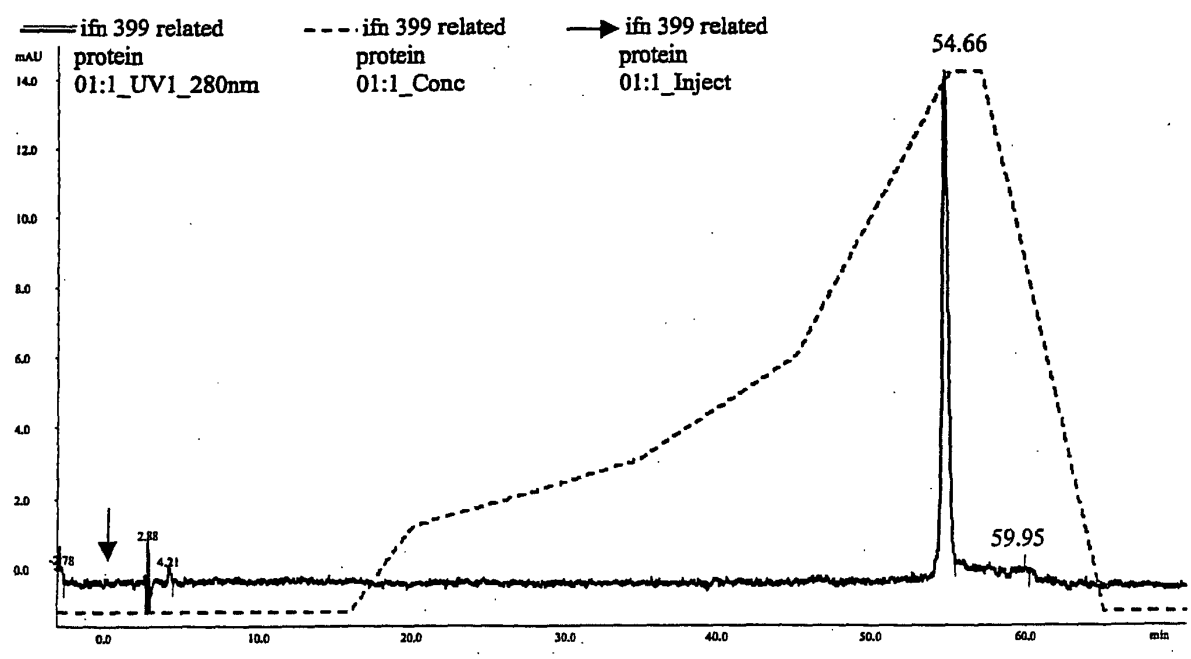


FIGURE 6b

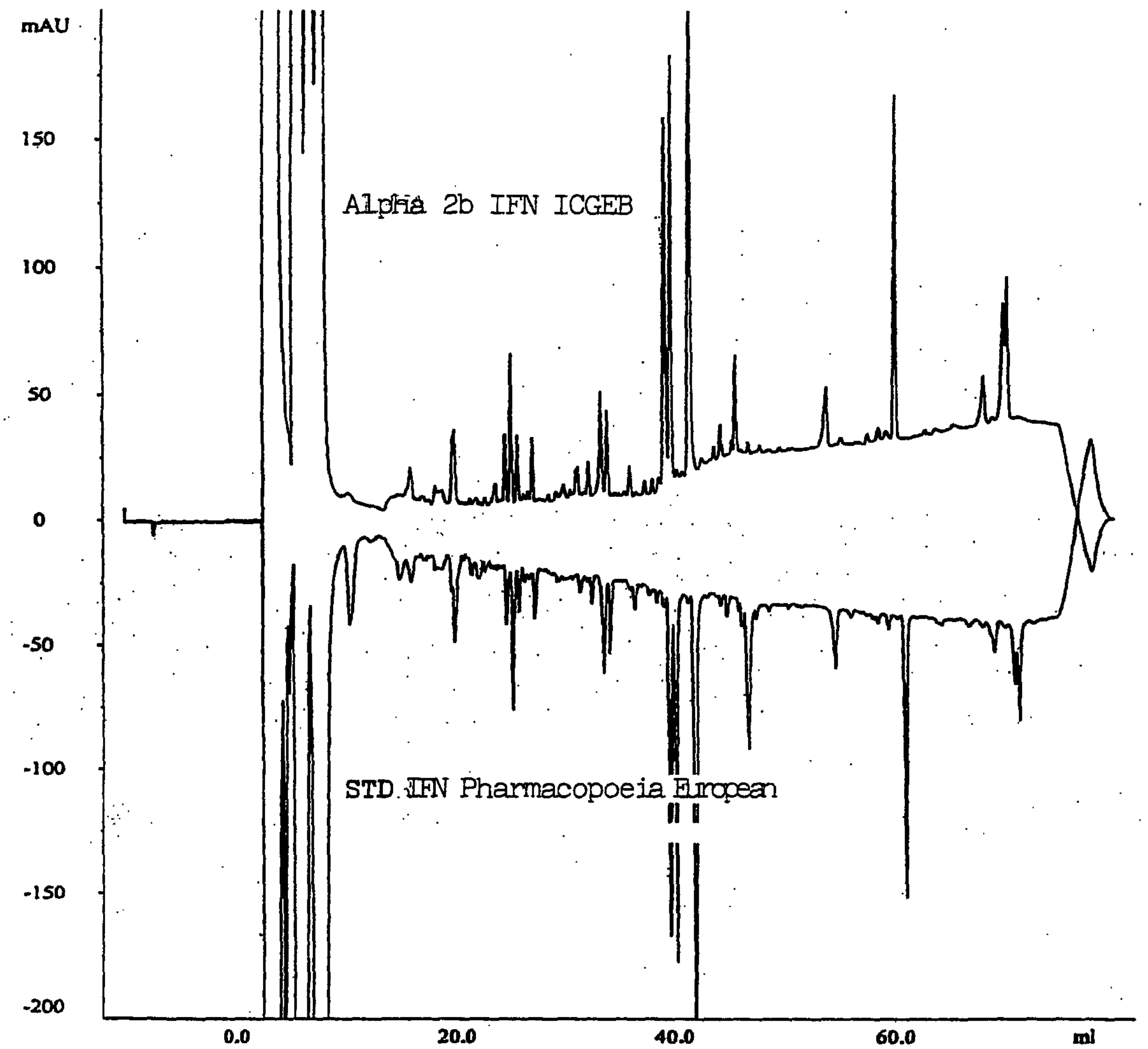
mAU
150
100
50
0
-50
-100
-150
-200
0.0
20.0
40.0
60.0
ml
Alpha 2b IFN ICGEB
STD IFN Pharmacopoeia European

FIGURE 7

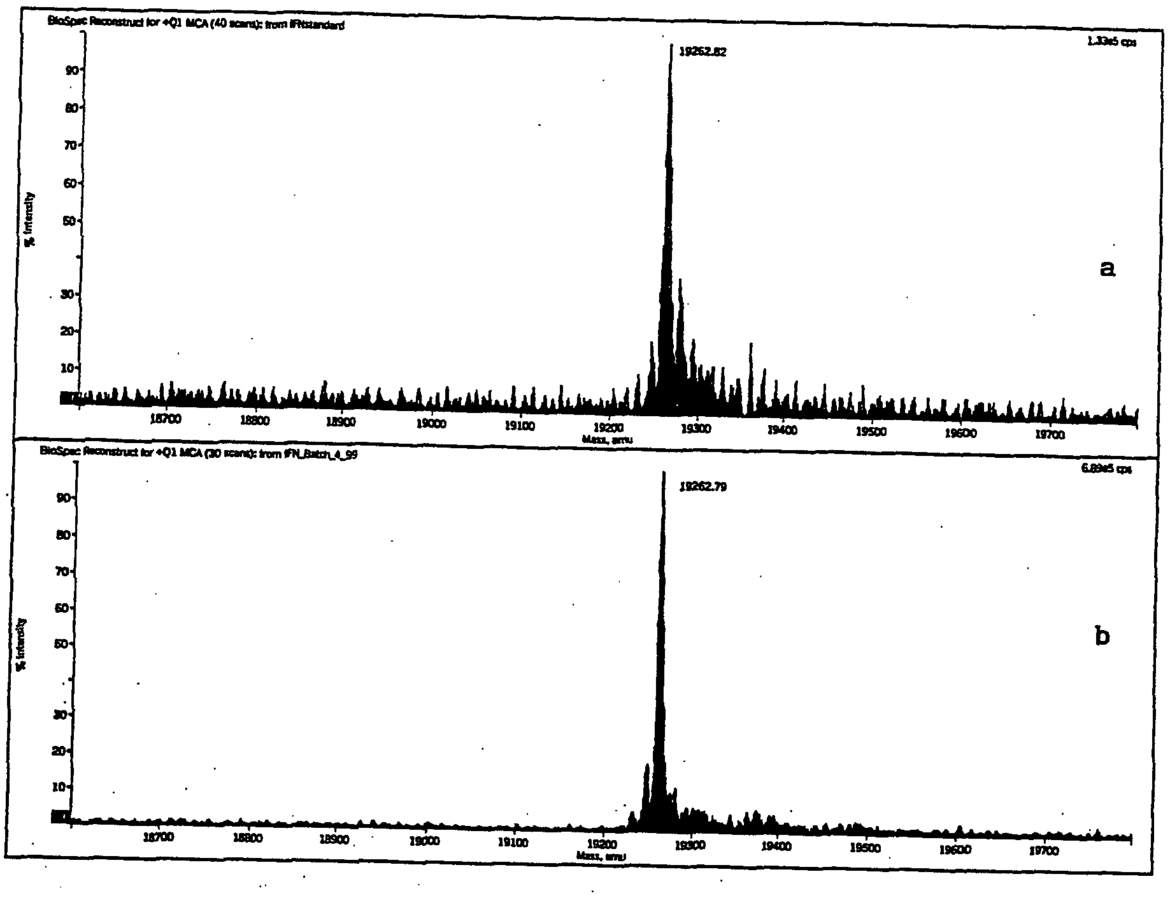
BioSpec Reconstruct for +Q1 MCA (40 scans): from IFNstandard
1.33e5 cps
19262.82
% Intensity
a
Mass, amu
BioSpec Reconstruct for +Q1 MCA (30 scans): from IFN_Batch_4_99
6.89e5 cps
19262.79
% Intensity
b
Mass, amu

FIGURE 8

European Patent Office

# EUROPEAN SEARCH REPORT

Application Number
EP 02 02 4919

| Category | Citation of document with indication, where appropriate, of relevant passages | Relevant to claim | CLASSIFICATION OF THE APPLICATION (Int.Cl.7) |
|---|---|---|---|
| D,X | DATABASE NCBI 'Online!<br>Database accession no. NM_000605<br>XP002232940<br>* abstract *<br>--- | 39-44 | C12N15/21<br>C07K14/56<br>C12P21/02<br>A61K38/21 |
| Y | BABU K R ET AL: "Production of interferon-alpha in high cell density cultures of recombinant Escherichia coli and its single step purification from refolded inclusion body proteins."<br>APPLIED MICROBIOLOGY AND BIOTECHNOLOGY,<br>vol. 53, no. 6, June 2000 (2000-06), pages 655-660, XP002232939<br>ISSN: 0175-7598<br>* the whole document *<br>--- | 1-46 | |
| Y | ARORA D ET AL: "Method for increasing the yield of properly folded recombinant human gamma interferon from inclusion bodies"<br>JOURNAL OF BIOTECHNOLOGY, ELSEVIER SCIENCE PUBLISHERS, AMSTERDAM, NL,<br>vol. 52, no. 2,<br>10 December 1996 (1996-12-10), pages 127-133, XP004095280<br>ISSN: 0168-1656<br>* the whole document *<br>--- | 1-46 | **TECHNICAL FIELDS SEARCHED (Int.Cl.7)**<br>C07K<br>C12N<br>C12P<br>A61K |
| D,Y | EP 0 679 718 A (HOFFMANN LA ROCHE)<br>2 November 1995 (1995-11-02)<br>* the whole document *<br>--- | 1-46 | |
| Y | EP 0 110 302 A (HOFFMANN LA ROCHE)<br>13 June 1984 (1984-06-13)<br>* page 1 - page 3 *<br>* page 7 - page 8 *<br>* page 15 - page 17 *<br>---<br>-/-- | 1-4,<br>11-14,<br>34,36-46 | |

The present search report has been drawn up for all claims

| Place of search | Date of completion of the search | Examiner |
|---|---|---|
| THE HAGUE | 6 March 2003 | Schneider, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 02 4919

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | US 4 485 038 A (CHADHA KAILASH C ET AL) 27 November 1984 (1984-11-27)<br>* column 7 *<br>* column 9 *<br>* column 12 *<br>--- | 19,21, 22,26, 34,36-38 | |
| D,Y | US 4 765 903 A (CLARK JR ARTHUR D ET AL) 23 August 1988 (1988-08-23)<br>* column 3 *<br>* column 6 - column 7 *<br>--- | 25,31,35 | |
| A | PATENT ABSTRACTS OF JAPAN vol. 003, no. 109 (C-058), 12 September 1979 (1979-09-12) & JP 54 089011 A (GREEN CROSS CORP:THE), 14 July 1979 (1979-07-14)<br>* abstract *<br>----- | | |

TECHNICAL FIELDS SEARCHED (Int.Cl.7)

The present search report has been drawn up for all claims

| Place of search | Date of completion of the search | Examiner |
|---|---|---|
| THE HAGUE | 6 March 2003 | Schneider, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 02 4919

This annex lists the patent family membersrelating to the patent documents cited in the above-mentioned European search report.
The members are as contained in the European Patent Office EDP file on
The European Patent Office is in no way liable for these particulars which are merely given for the purpose of information.

06-03-2003

| Patent document cited in search report | | Publication date | Patent family member(s) | | | Publication date |
|---|---|---|---|---|---|---|
| EP 0679718 | A | 02-11-1995 | EP | 0679718 | A2 | 02-11-1995 |
| | | | EP | 1104809 | A1 | 06-06-2001 |
| | | | AT | 205256 | T | 15-09-2001 |
| | | | DE | 69522492 | D1 | 11-10-2001 |
| | | | DE | 69522492 | T2 | 23-05-2002 |
| | | | DK | 679718 | T3 | 22-10-2001 |
| | | | ES | 2161793 | T3 | 16-12-2001 |
| | | | JP | 2859162 | B2 | 17-02-1999 |
| | | | JP | 7322887 | A | 12-12-1995 |
| | | | US | 6005075 | A | 21-12-1999 |
| EP 0110302 | A | 13-06-1984 | US | 4432895 | A | 21-02-1984 |
| | | | EP | 0110302 | A2 | 13-06-1984 |
| | | | JP | 59106428 | A | 20-06-1984 |
| US 4485038 | A | 27-11-1984 | NONE | | | |
| US 4765903 | A | 23-08-1988 | AT | 78698 | T | 15-08-1992 |
| | | | CA | 1329307 | A1 | 03-05-1994 |
| | | | DE | 3873353 | D1 | 03-09-1992 |
| | | | DE | 3873353 | T2 | 18-03-1993 |
| | | | EP | 0396555 | A1 | 14-11-1990 |
| | | | JP | 3500410 | T | 31-01-1991 |
| | | | WO | 8903225 | A1 | 20-04-1989 |
| JP 54089011 | A | 14-07-1979 | JP | 57004236 | B | 25-01-1982 |

EPO FORM P0459

For more details about this annex : see Official Journal of the European Patent Office, No. 12/82